(19) Europäisches Patentamt
European Patent Office
Office européen des brevets

(11) **EP 2 627 622 B1**

(12) **EUROPÄISCHE PATENTSCHRIFT**

(45) Veröffentlichungstag und Bekanntmachung des Hinweises auf die Patenterteilung:
**04.03.2015  Patentblatt 2015/10**

(51) Int Cl.:
*C07C 47/22* *(2006.01)*    *C07C 45/35* *(2006.01)*

(21) Anmeldenummer: **11776727.7**

(86) Internationale Anmeldenummer:
**PCT/EP2011/067887**

(22) Anmeldetag: **13.10.2011**

(87) Internationale Veröffentlichungsnummer:
**WO 2012/049246 (19.04.2012 Gazette 2012/16)**

(54) **VERFAHREN ZUM LANGZEITBETRIEB EINER HETEROGEN KATALYSIERTEN PARTIELLEN GASPHASENOXIDATION VON PROPEN ZU ACROLEIN**

METHOD FOR LONG-TERM OPERATION OF A HETEROGENEOUSLY CATALYZED PARTIAL GAS PHASE OXIDATION OF PROPENE TO OBTAIN ACROLEIN

PROCÉDÉ POUR RÉALISER, SUR UNE LONGUE DURÉE, L'OXYDATION EN PHASE GAZEUSE PARTIELLE À CATALYSE HÉTÉROGÈNE DE PROPÈNE EN ACROLÉINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.10.2010   US 393370 P**
**15.10.2010   DE 102010048405**

(43) Veröffentlichungstag der Anmeldung:
**21.08.2013   Patentblatt 2013/34**

(73) Patentinhaber: **BASF SE**
**67056 Ludwigshafen (DE)**

(72) Erfinder:
 • **MACHT, Josef**
  **68159 Mannheim (DE)**
 • **MÜLLER-ENGEL, Klaus Joachim**
  **76297 Stutensee (DE)**
 • **ROSOWSKI, Frank**
  **68239 Mannheim (DE)**

(56) Entgegenhaltungen:
**WO-A1-2007/082827     DE-A1-102010 048 405**

**Beschreibung**

Vorliegende Erfindung betrifft ein Verfahren zum Langzeitbetrieb einer heterogen katalysierten partiellen Gasphasenoxidation von Propen zu Acrolein, bei dem man ein Propen, molekularen Sauerstoff und wenigstens ein Inertgas enthaltendes Reaktionsgaseingangsgemisch, das den molekularen Sauerstoff und das Propen in einem molaren Verhältnis $O_2 : C_3H_6 \geq 1$ enthält, mit der Maßgabe durch ein Katalysatorfestbett, dessen Aktivmasse wenigstens ein die Elemente Mo, Fe und Bi enthaltendes Multimetalloxid ist, führt, dass

- das Katalysatorfestbett in zwei räumlich aufeinanderfolgenden (und in der Regel aneinandergrenzenden) Temperaturzonen A, B angeordnet ist (sich über zwei räumlich aufeinanderfolgende Temperaturzonen A, B erstreckt),

- sowohl die Temperatur $T^A$ der Temperaturzone A, als auch die Temperatur $T^B$ der Temperaturzone B eine Temperatur im Temperaturbereich von 280 bis 420°C ist,

- das Reaktionsgaseingangsgemisch die Temperaturzonen A, B in der zeitlichen Abfolge "erst A" und "dann B" durchströmt, wobei sich die Temperaturzone A bis zu einem Umsatz $U^A$ des im Reaktionsgaseingangsgemisch enthaltenen Propens im Bereich von 45 bis 85 mol-% erstreckt und sich in der Temperaturzone B der Umsatz des Propens auf einen Wert $U^B \geq 90$ mol-% erhöht,

- beim einmaligen Durchgang des Reaktionsgaseingangsgemischs durch das gesamte Katalysatorfestbett die Selektivität der Acroleinbildung, bezogen auf umgesetztes Propen,
$\geq 80$ mol-% beträgt,

- die Belastung des Katalysatorfestbetts mit dem im Reaktionsgaseingangsgemisch enthaltenen Propen $\geq 140$ Nl Propen/l Katalysatorfestbett·h beträgt,

- die Temperaturen $T^A$ und $T^B$ nach erfolgter frischer Beschickung des Katalysatorfestbetts so beschaffen sind, dass die Differenz $\Delta T^{BA} = T^B - T^A > 0$°C beträgt, und

- man anschließend mit zunehmender Betriebsdauer, um der Minderung der Qualität des Katalysatorfestbetts entgegenzuwirken, wenigstens eine der beiden Temperaturen $T^A$, $T^B$ erhöht.

Unter einem sich unter den Bedingungen der heterogen katalysierten Gasphasen-Partialoxidation von Propen zu Acrolein im Wesentlichen inert verhaltenden Verdünnungsgas (in dieser Schrift auch "Inertgas" genannt) werden in dieser Schrift solche Verdünnungsgase verstanden, deren Bestandteile unter den Bedingungen der heterogen katalysierten Gasphasen-Partialoxidation (oder auch partiellen Gasphasenoxidation) - jeder Bestandteil für sich betrachtet - zu mehr als 95 mol-%, vorzugsweise zu mehr als 97 mol-% und besonders bevorzugt zu mehr als 99 mol--% unverändert erhalten bleiben.

Unter der Belastung eines einen Reaktionsschritt katalysierenden Katalysatorfestbetts mit Reaktionsgasgemisch (z.B. Reaktionsgaseingangsgemisch) wird die Menge an Reaktionsgasgemisch in Normlitern (= Nl; das Volumen in Litern, das die entsprechende Reaktionsgasgemischmenge bei Normalbedingungen, d.h., bei 0°C und 1 atm (1013,25 hPa) einnehmen würde) verstanden, die dem Katalysatorfestbett, bezogen auf das (Schütt)Volumen seiner Schüttung zugeführt wird ($\rightarrow$ Einheit = Nl/l·h). Die Belastung kann auch nur auf einen Bestandteil des Reaktionsgasgemischs bezogen sein. Dann ist es die Nl-Volumenmenge dieses Bestandteils, die dem Katalysatorfestbett, bezogen auf das Volumen seiner Schüttung, pro Stunde zugeführt wird.

Das Reaktionsgaseingangsgemisch enthält u.a. deshalb Inertgas, um das Reaktionsgasgemisch außerhalb des Explosionsbereichs zu halten.

Das eingangs dieser Schrift beschriebene Verfahren der heterogen katalysierten partiellen Gasphasenoxidation von Propen zu Acrolein an einem frisch beschickten Katalysatorfestbett (d.h., an dem Katalysatorfestbett wurde noch keine Partialoxidation durchgeführt; es enthält neu hergestellte, frisch gefertigte Katalysatoren) ist z.B. aus der WO 2004/085362 sowie aus dem in selbiger WO zitierten Stand der Technik grundsätzlich bekannt.

Es ist unter anderem als erste Oxidationsstufe bei der Herstellung von Acrylsäure durch zweistufige heterogen katalysierte Gasphasenoxidation ausgehend von Propen von Bedeutung. Acrylsäure ist ein bedeutendes Monomer, das als solches oder in Form seiner Alkylester zur Erzeugung von z.B. als Klebstoffe geeigneten oder Wasser absorbierenden Polymerisaten Verwendung findet. Acrolein ist ein bedeutendes Zwischenprodukt.

Dem Katalysatorfestbett kommt dabei die Aufgabe zu, zu bewirken, dass die gewünschte Partialoxidation des Propens, z.B. gegenüber der vollständigen Oxidation des Propens zu Kohlenoxiden und Wasser, bevorzugt abläuft.

Die chemische Umsetzung erfolgt, wenn das Reaktionsgasgemisch das Katalysatorfestbett durchströmt, während der Verweilzeit des Reaktionsgasgemischs in selbigem.

Die heterogen katalysierte partielle Gasphasenoxidation von Propen zu Acrolein verläuft ausgeprägt exotherm. Infolge einer Vielfalt möglicher Parallel- und/oder Folgereaktionen ist im Hinblick auf eine möglichst selektive Umsetzung des Propens zum Acrolein die alleinige Maßnahme einer Katalysatormitverwendung normalerweise nicht ausreichend. Vielmehr ist es für eine möglichst selektive Durchführung der heterogen katalysierten Gasphasen-Partialoxidation des Propens zum Acrolein im Katalysatorfestbett, bei gleichzeitig angestrebtem angemessenem Umsatz des Propens, zusätzlich erforderlich, den Verlauf der Reaktionstemperatur bzw. den Verlauf der Temperatur des Katalysatorfestbetts (im Katalysatorfestbett) in Strömungsrichtung des Reaktionsgasgemischs in gewissem Umfang zu lenken (vgl. z.B. EP-A 700714).

Gemäß den Lehren des Standes der Technik hat es sich diesbezüglich insbesondere im Fall erhöhter Propenbelastungen des Katalysatorfestbetts in der Regel als vorteilhaft erwiesen, ein frisch beschicktes Katalysatorfestbett in zwei räumlich aufeinanderfolgenden Temperaturzonen A, B aufzuschütten, deren Temperaturen $T^A$ und $T^B$ so beschaffen sind, dass die Differenz $\Delta T^{BA} = T^B - T^A > 0°C$ beträgt, und das Propen und den molekularen Sauerstoff sowie das wenigstens eine Inertgas enthaltende Reaktionsgaseingangsgemisch so durch das Katalysatorfestbett zu führen, dass das Reaktionsgaseingangsgemisch die Temperaturzonen A, B in der zeitlichen Abfolge "erst A" und "dann B" durchströmt, wobei die Länge der Temperaturzone A so bemessen ist, dass sie sich bis zu einem Umsatz $U^A$ des im Reaktionsgaseingangsgemisch (das Gasgemisch, das dem Katalysatorfestbett zugeführt wird) enthaltenen Propens im Bereich von 45 bis 85 mol-% erstreckt und die Länge der Temperaturzone B so bemessen ist, dass sich in der Temperaturzone B (beim Durchgang des Reaktionsgasgemischs durch die Reaktionszone B) der Umsatz des Propens auf einen Wert $U^B$ ≥ 90 mol-% erhöht (vgl. z.B. EP-A 1159244).

In der Praxis werden die Temperaturzonen A, B dabei in der Regel so verwirklicht, dass man das Katalysatorfestbett in einen Reaktionsraum einbringt (z.B. der Innenraum eines (Reaktions)Rohres), um den in zwei voneinander im Wesentlichen getrennten, in Strömungsrichtung des Reaktionsgasgemischs räumlich aufeinanderfolgenden (und in der Regel aneinandergrenzenden) Abschnitten A, B aus Gründen der Temperaturregelung (Wärmeabfuhr) jeweils ein fluider (vorzugsweise flüssiger) Wärmeträger (ein Wärmeaustauschmittel) geführt bzw. gleitet (ein- und aus-) wird, der die materielle Einhüllende des Reaktionsraums (die Wand des Reaktionsraums) entlang des jeweiligen Abschnitts A bzw. B berührt (sich mit selbiger in Kontakt befindet). Der im Abschnitt A geführte Wärmeträger wird dabei normalerweise mit der Temperatur $T^A$ und der im Abschnitt B geführte Wärmeträger wird dabei normalerweise mit der Temperatur $T^B$ zugeführt. Die Gesamtwärmekapazität des geführten Wärmeträgerstroms ist dabei normalerweise sehr viel größer als die Gesamtwärmekapazität des Reaktionsgasgemischstroms. Hinsichtlich des Wärmeübergangskoeffizienten (von der materiellen Einhüllenden zum fluiden Wärmeträger) wird wie in der EP-A 700714 implizit und in der EP-A 1547994 explizit beschrieben verfahren.

Unter der Temperatur einer Temperaturzone wird dabei im Stand der Technik, wie auch in dieser Schrift, die Temperatur des in der Temperaturzone befindlichen Teils des Katalysatorfestbetts (bzw. Teils der Festbettkatalysatorschüttung) bei Ausübung des erfindungsgemäßen Verfahrens, jedoch in fiktiver Abwesenheit der chemischen Reaktionswärme verstanden.

Diese Temperatur entspricht normalerweise im Wesentlichen derjenigen Temperatur, mit welcher der zugehörige Wärmeträger der jeweiligen Temperaturzone zuströmt.

Beispielsweise und anwendungstechnisch besonders einfach kann sich das Katalysatorfestbett (die Festbettkatalysatorschüttung) in den Kontaktrohren (Reaktionsrohren) eines sogenannten Zweizonenrohrbündelreaktors befinden (aufgeschüttet sein), wie er z.B. in den DE-A's 19910508, 19948523, 19910506 und 19948241 sowie in den Schriften WO 2004/085362, WO 2007/082827, WO 2004/085370, WO 2004/085369, WO 2004/085363, WO 2004/085365, WO 2004/007064 und WO 2004/085367 sowie in der in den vorgenannten Schriften zitierten Sekundärliteratur beschrieben ist.

Eine bevorzugte Variante eines erfindungsgemäß einsetzbaren Zweizonenrohrbündelreaktors offenbart die DE-C 2830765. Aber auch die in der DE-C 2513405, der US-A 3,147,084, der DE-A 2201528, der EP-A 383224 und der DE-A 2903218 offenbarten Zweizonenrohrbündelreaktoren sind für eine Durchführung der relevanten Verfahrensweise geeignet.

D.h., in einfacher Weise befindet sich die erfindungsgemäß zu verwendende Festbettkatalysatorschüttung (das Katalysatorfestbett) in den Reaktionsrohren eines Vielkontaktrohr-Festbettreaktors (Rohrbündelreaktors) und um die Reaktionsrohre werden zwei voneinander im Wesentlichen räumlich getrennte Temperiermedien (fluide Wärmeübertrager, Wärmeaustauschmittel) wie z.B. ionische Flüssigkeiten, Wasser (Dampf), Salzschmelzen oder flüssige Metalle geführt (geleitet; ein- und aus-). Der Rohrabschnitt, über den sich das jeweilige Salzbad bzw. Metallbad erstreckt, repräsentiert eine Temperaturzone.

Entsprechende Temperaturzonen können auch in Thermoblechplattenreaktoren, wie sie z.B. die WO 2005/009608 offenbart, oder in Wärmetauscherplattenreaktoren, wie sie z.B. die EP-A 1577001 beschreibt, eingerichtet werden.

Zusätzlich zu den vorstehend beschriebenen externen Maßnahmen der Temperaturlenkung tragen auch die bereits erwähnten, im Reaktionsgaseingangsgemisch mitverwendeten inerten Verdünnungsgase, die mit ihrer Wärmekapazität frei werdende Reaktionswärme zu absorbieren vermögen (interne Maßnahmen der Temperaturlenkung) zur Lenkung des Reaktionstemperaturverlaufs im Katalysatorfestbett bei.

Eines der häufigsten mitverwendeten inerten Verdünnungsgase ist molekularer Stickstoff, der automatisch immer dann zu Anwendung kommt, wenn als Sauerstoffquelle für die heterogen katalysierte Gasphasen-Partialoxidation Luft verwendet wird.

Ein anderes vielfach mitverwendetes inertes Verdünnungsgas ist wegen seiner allgemeinen Verfügbarkeit und vorteilhaften molaren Wämekapazität Wasserdampf.

Andere in typischer Weise mitverwendete inerte Verdünnungsgase sind Edelgase (z.B. He, Ar, Ne) oder die Kohlenoxide $CO_2$ und/oder CO.

Als Inertgase mit vergleichsweise großer molarer Wärmekapazität kommen für die heterogen katalysierte partielle Gasphasenoxidation von Propen zu Acrolein unter anderem auch gesättigte Kohlenwasserstoffe wie z.B. n-Propan und Ethan in Betracht. Vielfach wird auch Kreisgas als inertes Verdünnungsgas mitverwendet (vgl. EP-A 1180508). Als Kreisgas wird das Restgas bezeichnet, das nach einer einstufigen oder mehrstufigen (die heterogen katalysierte Partialoxidation von Propen zu Acrolein kann z.B. auch als lediglich erste Reaktionsstufe einer zweistufigen heterogen katalysierten Partialoxidation von Propen zu Acrylsäure angewendet werden) heterogen katalysierten Gasphasen-Partialoxidation des Propens zu Acrolein bzw. Acrylsäure dann verbleibt, wenn man aus dem Produktgasgemisch das Zielprodukt (Acrolein bzw. Acrylsäure) mehr oder weniger selektiv (z.B. durch Absorption in ein geeignetes Lösungsmittel, oder durch fraktionierende Kondensation, oder durch eine Überlagerung von Absorption und Kondensation) abgetrennt hat (vgl. z.B. WO 2007/082827, Seite 6 ff).

Im Regelfall besteht es überwiegend aus den für die Partialoxidation verwendeten inerten Verdünnungsgasen sowie aus bei der Partialoxidation üblicherweise als Nebenprodukt gebildetem oder als Verdünnungsgas zugesetztem Wasserdampf und durch unerwünschte vollständige Oxidation als Nebenreaktion gebildeten Kohlenoxiden. Teilweise enthält es noch geringe Mengen an bei der Partialoxidation nicht verbrauchtem molekularem Sauerstoff (Restsauerstoff) und/oder an nicht umgesetztem Propen und/oder nicht umgesetztem Zwischenprodukt Acrolein.

Trotz der beschriebenen externen und internen Maßnahmen zur Lenkung (Steuerung) der Reaktionstemperatur bzw. der Temperatur des Katalysatorfestbetts (die beiden vorgenannten Temperaturen sind im Wesentlichen identisch) sind die Temperaturen der Temperaturzonen A, B normalerweise von der in Strömungsrichtung längs des Katalysatorfestbetts jeweils herrschenden Reaktionstemperatur (= die jeweilige Temperatur des Reaktionsgasgemischs) bzw. jeweils vorliegenden effektiven Temperatur des Katalysatorfestbetts (sie entspricht im Wesentlichen der auf gleicher Höhe vorliegenden Reaktionstemperatur) verschieden. Die effektive Temperatur des Katalysatorfestbetts ist dabei die tatsächliche Temperatur des Katalysatorfestbetts, die sowohl den Einfluss des außerhalb des Reaktionsraums geführten fluiden Wärmeträgers, als auch die Reaktionswärme der Partialoxidation beinhaltet (wohingegen der Begriff "Temperatur der Temperaturzone", wie bereits gesagt, den Einfluss der Reaktionswärme der Partialoxidation ausklammert). Die Temperatur einer Temperaturzone ist im Unterschied zur effektiven Temperatur des Katalysatorfestbetts in Strömungsrichtung längs desselben normalerweise im Wesentlichen konstant. Ist die Temperatur einer Temperaturzone nicht völlig konstant, so meint der Begriff "Temperatur einer Temperaturzone" hier den (Zahlen) Mittelwert der Temperatur über die Temperaturzone. Die Temperierung der einzelnen Temperaturzonen erfolgt im Wesentlichen unabhängig voneinander. Normalerweise ist die effektive Temperatur des Katalysatorfestbetts auf der jeweiligen Betthöhe größer als die Temperatur der zugehörigen Temperaturzone.

Von Bedeutung im vorgenannten Zusammenhang ist, dass die Temperatur des Reaktionsgasgemischs (und damit auch die effektive Temperatur des Katalysatorfestbetts) beim Durchschreiten des Katalysatorfestbetts in Strömungsrichtung des Reaktionsgasgemischs in der jeweiligen Temperaturzone üblicherweise einen Höchstwert durchläuft bzw. von einem solchen Maximalwert ausgehend abfällt (der sogenannte Heißpunktwert $T^{maxA}$ (in der Temperaturzone A) bzw. $T^{maxB}$ (in der Temperaturzone B)). Die Differenz zwischen Heißpunktwert und der Temperatur der zugehörigen Temperaturzone wird als Heißpunktausdehnung $\Delta T^{HB}_A$ (in der Temperaturzone A) bzw. $\Delta T^{HB}_B$ (in der Temperaturzone B) bezeichnet.

Eine Ursache dafür ist, dass die Reaktantenkonzentration im Reaktionsgasgemisch am Eingang (Eintritt) des Reaktionsgasgemischs in das Katalysatorfestbett am höchsten ist, was dort besonders hohe Reaktionsgeschwindigkeiten bedingt, mit denen pro Zeiteinheit eine besonders hohe Reaktionswärmeentwicklung einhergeht (beim Eintritt in das Katalysatorfestbett weist das Reaktionsgasgemisch (= das Reaktionsgaseingangsgemisch) in der Regel im Wesentlichen die Temperatur der Temperaturzone A auf).

Eine andere Ursache dafür ist der endliche Wärmeübergang vom Reaktionsgasgemisch auf den Wärmeträger.

Gemäß der Lehre des Standes der Technik werden beim frisch beschickten Katalysatorfestbett die allgemeinen Verfahrensbedingungen in der Regel vorteilhaft so gewählt, dass $T^{maxA} - T^{maxB} \geq 0°C$ beträgt (vgl. WO 2004/085362, WO 2004/085370 und WO 2004/085363).

Ferner werden gemäß den Lehren des zitierten Standes der Technik beim frisch beschickten Katalysatorfestbett die allgemeinen Verfahrensbedingungen normalerweise so gewählt, dass sowohl $\Delta T^{HB}_B$ als auch $\Delta T^{HB}_A$ in der Regel 90°C nicht überschreiten. Meist betragen diese Temperaturunterschiede ≥ 3°C und ≤ 80°C, oder ≤ 70°C, häufig ≥ 5°C und ≤ 60°C, bzw. ≤ 50°C. Anwendungstechnisch zweckmäßig beträgt $\Delta T^{HB}_A$ 40 bis 90°C, bzw. 40 bis 80°C.

Weiterhin beträgt beim frisch beschickten Katalysatorfestbett (vorzugsweise gleichzeitig) die Änderung von $\Delta T^{HB}_A$ bzw. $\Delta T^{HB}_B$ bei Erhöhung der Temperatur der zugehörigen Temperaturzone um +1°C normalerweise (vgl. die gewürdigten Schriften des Standes der Technik) ≤ +9°C, vorzugsweise ≤ +7°C, oder ≤ +5°C bzw. ≤ +3°C, aber > +0°C (vgl. z.B. EP-A 1106598).

Die Platzierung sowohl von $T^A$ als auch $T^B$ im Temperaturbereich von 280°C bis 420°C gewährleistet beim einmaligen Durchgang des Reaktionsgasgemischs durch das Katalysatorfestbett (insbesondere durch das frisch beschickte Katalysatorfestbett) wirtschaftliche Propenumsätze (typischerweise ≥ 90 mol-%).

Der Arbeitsdruck kann bei der heterogen katalysierten partiellen Gasphasenoxidation von Propen zu Acrolein am Katalysatorfestbett sowohl unter 1 atm (= 1,0133.10⁵ Pa) oder über 1 atm liegen. In der Regel liegt er im Bereich von ≥ 1 bis 20, bzw. bis 10 atm, oder bis 5 atm. Ein Arbeitsdruck von 100 atm wird üblicherweise nicht überschritten.

Die Katalysatoren des Katalysatorfestbetts für die heterogen katalysierte partielle Gasphasenoxidation von Propen zu Acrolein weisen in der Regel als Aktivmasse wenigstens ein die Elemente Mo, Fe und Bi enthaltendes Multimetalloxid auf (vgl. z.B. WO 2004/085362).

Es ist nun allgemein bekannt, dass eine wie eingangs beschriebene heterogen katalysierte Gasphasen-Partialoxidation von Propen zu Acrolein nach erfolgter frischer Beschickung des Reaktors mit einem frischen Katalysatorfestbett im Wesentlichen kontinuierlich und über einen längeren Zeitraum unter im Wesentlichen unveränderten Bedingungen unter Beibehalt des Katalysatorfestbetts (d.h., ohne das Katalysatorfestbett zu wechseln, an ein und demselben Katalysatorfestbett) betrieben werden kann.

Allerdings verliert das Katalysatorfestbett dabei mit zunehmender Betriebszeit normalerweise an Qualität (vgl. z.B. DE-A 102004025445). In der Regel verschlechtert sich vor allem die volumenspezifische Aktivität des Katalysatorfestbetts (unter ansonsten unveränderten Verfahrensbedingungen nimmt der auf den einmaligen Durchgang des Reaktionsgasgemischs durch das Katalysatorfestbett bezogene Propenumsatz mit zunehmender Betriebszeit ab, was die beabsichtigte Raum-Zeit-Ausbeute einer Acroleinproduktionsanlage an Acrolein bzw. an Acrolein und Acrylsäure mindert). Häufig leidet auch die Selektivität der Acroleinbildung bzw. der Gesamtzielproduktbildung an Acrolein und Acrylsäure.

Die EP-A 1106598 und die DE-A 10351269 versuchen der vorgenannten Entwicklung im Langzeitbetrieb der heterogen katalysierten Gasphasen-Partialoxidation des Propens zum Acrolein dadurch Rechnung zu tragen, dass die Temperatur der Temperaturzone, in der sich das Katalysatorfestbett befindet, und mit dieser die Temperatur des Katalysatorfestbetts, unter ansonsten weitgehend gleichbleibenden Betriebsbedingungen nach und nach erhöht wird, um den Propenumsatz bei einmaligem Durchgang des Reaktionsgasgemischs durch das Katalysatorfestbett im Wesentlichen beizubehalten (dabei kann, wie z.B. die WO 2004/085369, die DE-A 10351269, die DE-A 10350812 und die EP-A 614872 empfehlen, der Qualitätsminderung des Katalysatorfestbetts im Langzeitbetrieb zwischendurch zusätzlich dadurch entgegengetreten werden, dass das Katalysatorfestbett von Zeit zu Zeit regeneriert wird; dazu wird das Verfahren der heterogen katalysierten Festbett-Gasphasen-Partialoxidation von Propen zu Acrolein (bzw. zu Acrolein und Acrylsäure) unterbrochen und z.B. ein heißes Gemisch aus molekularem Sauerstoff und Inertgas durch das Katalysatorfestbett geführt; auf diese Weise gelingt es, einen reversiblen Anteil der Minderung der Qualität (der Alterung) des Katalysatorfestbetts wieder rückgängig zu machen). Eine solche Regenerierung kann auch gemäß der DE-A 102004008573, bzw. der WO 05/082517 erfolgen. Beispielsweise kann eine solche Regenerierung immer dann durchgeführt werden, wenn die Partialoxidation z.B. deshalb unterbrochen wird, weil das Reaktionsgasgemisch versehentlich eine explosionstechnisch gegebenenfalls nur schwer beherrschbare Zusammensetzung angenommen hat.

Nachteilig an den Lehren der EP-A 1106598 und der DE-A 10351269 ist jedoch, dass sie eine synchrone Erhöhung der Temperatur in den beiden Temperaturzonen A, B nahe legen. D.h., $T^A$ und $T^B$ werden jeweils im gleichen Ausmaß (um gleich viel °C) erhöht.

Eine solche Verfahrensweise ist zwar gegenüber einer Verfahrensweise ohne Erhöhung der Temperatur des Katalysatorfestbetts vorteilhaft und kann auch grundsätzlich bei der eingangs dieser Schrift beschriebenen heterogen katalysierten Partialoxidation von Propen zu Acrolein (bzw. zu Acrolein und Acrylsäure) vorteilhaft angewendet werden.

Sie ist jedoch insofern nachteilig, als sie zwar unter ansonsten unveränderten Betriebsbedingungen den Beibehalt des angestrebten Propenumsatzes (bezogen auf einmaligen Durchgang des Reaktionsgasgemischs durch das Katalysatorfestbett) gewährleistet, dies allerdings normalerweise zum Preis einer verringerten Selektivität der Acroleinbildung bzw. der Gesamtzielproduktbildung an Acrolein und Acrylsäure (vgl. WO 2007/082827).

Die WO 2007/082827 empfiehlt daher, den Langzeitbetrieb einer wie eingangs beschriebenen heterogen katalysierten partiellen Gasphasenoxidation von Propen zu Acrolein (dies ist immer auch eine Gasphasenpartialoxidation von Propen zu Acrolein und Acrylsäure) so durchzuführen, dass man, um der Minderung der Qualität des Katalysatorfestbetts entgegenzuwirken, mit zunehmender Betriebsdauer die Temperatur der Temperaturzone A, die zunächst (nach erfolgter frischer Beschickung des Katalysatorfestbetts) eine tiefere Temperatur als die Temperaturzone A aufwies, erhöht, und gleichzeitig die Temperatur der Temperaturzone B so weiterentwickelt, dass sich die Differenz $\Delta T^{BA} = T^B$-

$T^A$ zwischen den Temperaturen der beiden Temperaturzonen $T^A$, $T^B$ mit zunehmender Betriebsdauer verringert und gegebenenfalls sogar das Vorzeichen (von + nach -) wechselt.

Nachteilig an der Lehre der WO 2007/082827 ist jedoch, dass die in ihr empfohlene Langzeitbetriebsweise die Standzeit des Katalysatorfestbetts frühzeitig beeinträchtigt. D.h., sie führt zu einer vergleichsweise ausgeprägten Beschleunigung der irreversiblen Alterung des Katalysatorfestbetts. Erreicht der Grad der irreversiblen Alterung des Katalysatorfestbetts den Punkt, ab dem selbst bei weiterer Erhöhung der Temperatur des Katalysatorfestbetts der auf einen Einmaldurchgang des Reaktionsgasgemischs durch das Katalysatorfestbett bezogene Umsatz des Propens nicht mehr, oder allenfalls noch auf Kosten einer signifikant verringerten Selektivität der Acroleinbildung bzw. der Gesamtzielproduktbildung an Acrolein und Acrylsäure aufrechterhalten werden kann, muss das Katalysatorfestbett aus Gründen der Wirtschaftlichkeit des Verfahrens wenigstens teilweise (vgl. DE-A 10232748) oder vollständig durch ein frisches Katalysatorfestbett ersetzt werden. Dieser Zeitpunkt wird als Endpunkt der Standzeit des Katalysatorfestbetts bezeichnet.

Die Aufgabe der vorliegenden Erfindung bestand daher darin, ein verbessertes Verfahren des Langzeitbetriebs einer wie eingangs dieser Schrift beschrieben in zwei Temperaturzonen durchgeführten heterogen katalysierten partiellen Gasphasenoxidation von Propen zu Acrolein zur Verfügung zu stellen, das bei einer verbesserten Standzeit des Katalysatorfestbetts über die Betriebsdauer auch eine befriedigende Selektivität der Acroleinbildung bzw. der Gesamtzielproduktbildung an Acrolein und Acrylsäure gewährleistet.

Demgemäß wird ein Verfahren zum Langzeitbetrieb einer heterogen katalysierten partiellen Gasphasenoxidation von Propen zu Acrolein (dies ist immer auch eine Gasphasenpartialoxidation von Propen zu Acrolein und Acrylsäure), bei dem man ein Propen, molekularen Sauerstoff und wenigstens ein Inertgas enthaltendes Reaktionsgaseingangsgemisch, das den molekularen Sauerstoff und das Propen in einem molaren Verhältnis $O_2 : C_3H_6 \geq 1$ enthält, mit der Maßgabe durch ein Katalysatorfestbett, dessen Aktivmasse wenigstens ein die Elemente Mo, Fe und Bi enthaltendes Multimetalloxid ist, führt, dass

- das Katalysatorfestbett in zwei räumlich aufeinanderfolgenden Temperaturzonen A, B angeordnet ist,

- sowohl die Temperatur $T^A$ der Temperaturzone A, als auch die Temperatur $T^B$ der Temperaturzone B eine Temperatur im Temperaturbereich von 280 bis 420°C ist,

- das Reaktionsgaseingangsgemisch die Temperaturzonen A, B in der zeitlichen Abfolge "erst A" und "dann B" durchströmt, wobei sich die Temperaturzone A bis zu einem Umsatz $U^A$ des im Reaktionsgaseingangsgemisch enthaltenen Propens im Bereich von 45 bis 85 mol-% erstreckt und sich in der Temperaturzone B der Umsatz des Propens auf einen Wert $U^B \geq 90$ mol-% erhöht,

- beim einmaligen Durchgang des Reaktionsgaseingangsgemischs durch das gesamte Katalysatorfestbett die Selektivität der Acroleinbildung, bezogen auf umgesetztes Propen, $\geq 80$ mol-% beträgt,

- die Belastung des Katalysatorfestbetts mit dem im Reaktionsgaseingangsgemisch enthaltenen Propen $\geq 140$ Nl Propen/l·Katalysatorfestbett·h beträgt,

- die Temperaturen $T^A$ und $T^B$ nach erfolgter frischer Beschickung des Katalysatorfestbetts so beschaffen sind, dass die Differenz $\Delta T^{BA} = T^B - T^A > 0$°C beträgt, und

- man anschließend mit zunehmender Betriebsdauer, um der Minderung der Qualität des Katalysatorfestbetts entgegenzuwirken, wenigstens eine der beiden Temperaturen $T^A$, $T^B$ erhöht,

zur Verfügung gestellt, das dadurch gekennzeichnet ist, dass

- die Erhöhung der wenigstens einen der beiden Temperaturen $T^A$, $T^B$ so vorgenommen wird, dass sich die Differenz $\Delta T^{BA} = T^B - T^A$ mit zunehmender Betriebsdauer erhöht.

Mit Vorteil wird die Differenz $\Delta T^{BA} = T^B - T^A$ für die Durchführung des erfindungsgemäßen Verfahrens am frisch beschickten Katalysatorfestbett (d.h., zu Beginn des erfindungsgemäßen Verfahrens) $\geq 2$°C, vorzugsweise $\geq 3$°C und besonders bevorzugt $\geq 5$°C betragen. Üblicherweise beträgt die Differenz $\Delta T^{BA}$ für die Durchführung des erfindungsgemäßen Verfahrens am frisch beschickten Katalysatorfestbett $\leq 50$°C und vorzugsweise $\leq 40$°C.

Erfindungsgemäß günstige Differenzen $\Delta T^{BA} = T^B - T^A$ für die Durchführung des erfindungsgemäßen Verfahrens am frisch beschickten Katalysatorfestbett liegen im Bereich $\geq 5$°C und $\leq 35$°C, bzw. $\geq 5$°C und $\leq 30$°C, oder $\geq 5$°C und $\leq 25$°C, oder $\geq 10$°C und $\leq 25$°C bzw. $\geq 15$°C und $\leq 20$°C.

Im Regelfall geht mit der Einstellung der vorstehenden Differenzen $\Delta T^{BA}$ für die Durchführung des erfindungsgemäßen Verfahrens am frisch beschickten Katalysatorfestbett (d.h., zu Beginn des erfindungsgemäßen Verfahrens) eine Differenz $T^{maxA}$ - $T^{maxB}$ einher, die ≥ 0°C und ≤ 80°C, oder ≤ 70°C beträgt.

Anwendungstechnisch zweckmäßig wird bei der Durchführung des erfindungsgemäßen Verfahrens am frisch beschickten Katalysatorfestbett eine nicht zu ausgeprägte Differenz $T^{maxA}$ - $T^{maxB}$ angestrebt.

Günstige Differenzen $T^{maxA}$ - $T^{maxB}$ bei der Durchführung des erfindungsgemäßen Verfahrens am frisch beschickten Katalysatorfestbett (d.h., zu Beginn des erfindungsgemäßen Verfahrens) betragen daher ≥ 1°C und ≤ 60°C, oder ≤ 50°C, vorzugsweise ≥ 2°C und ≤ 40°C, mit Vorteil ≥ 3°C und ≤ 30°C, mit besonderem Vorteil ≥ 5°C oder ≥ 10°C und ≤ 25°C sowie ganz besonders bevorzugt ≥ 5°C und ≤ 20°C bzw. ≤ 15°C.

Kennzeichnend für die erfindungsgemäße Verfahrensweise ist, dass im Langzeitbetrieb der heterogen katalysierten partiellen Gasphasenoxidation des Propens zum Acrolein am selben Katalysatorfestbett, um der Minderung der Qualität des Katalysatorfestbetts entgegenzuwirken, wenigstens eine der beiden Temperaturen $T^A$, $T^B$ mit der Maßgabe erhöht wird, dass sich dabei die Differenz $\Delta T^{BA} = T^B - T^A$ mit zunehmender Betriebsdauer erhöht.

D.h., erfindungsgemäß ist so vorzugehen, dass, jeweils bezogen auf die selbe Betriebsdauer, $T^B$ im Langzeitbetrieb eine stärkere Erhöhung erfährt als $T^A$.

Mit anderen Worten ausgedrückt, kann im erfindungsgemäßen Langzeitbetrieb sowohl $T^B$ als auch $T^A$ erhöht werden.

Erfindungsgemäß vorteilhaft beträgt die Erhöhung von $T^B$ das 1,2- bis 5-fache der Erhöhung von $T^A$, jeweils bezogen auf die selbe Betriebsdauer im Langzeitbetrieb des erfindungsgemäßen Verfahrens.

D.h., günstige erfindungsgemäße Verfahren sind solche, bei denen die Erhöhung von $T^B$ das 1,5- bis 4-fache, oder das 1,5- bis 3-fache (z.B. das 2-fache) der Erhöhung von $T^A$, jeweils bezogen auf die selbe Betriebsdauer im Langzeitbetrieb des erfindungsgemäßen Verfahrens, beträgt.

Konsequenz der erfindungsgemäßen Verfahrensweise ist normalerweise, dass sich auch die Differenz $T^{maxA}$ - $T^{maxB}$ mit zunehmender Betriebsdauer verringert (im Vergleich zum Differenzwert $T^{maxA}$ - $T^{maxB}$ nach erfolgter frischer Beschickung des Katalysatorfestbetts).

Es hat sich jedoch als erfindungsgemäß vorteilhaft erwiesen, zu vermeiden, dass $T^{maxA}$ - $T^{maxB}$ im Langzeitbetrieb des erfindungsgemäßen Verfahrens negative Werte annimmt. Jedoch schließt die erfindungsgemäße Verfahrensweise ein "negativ" werden der Differenz $T^{maxA}$ - $T^{maxB}$ nicht aus. Erfindungsgemäß zweckmäßig wird man in der Regel deshalb so verfahren, dass die Differenz $T^{maxA}$ - $T^{maxB}$ im Langzeitbetrieb möglichst lange (z.B. innerhalb einer Betriebsdauer von wenigstens 6 Monaten, vorzugsweise innerhalb einer Betriebsdauer von wenigstens 12 Monaten, vorteilhaft innerhalb einer Betriebsdauer von wenigstens 18 Monaten, besonders vorteilhaft innerhalb einer Betriebsdauer von wenigstens 24 Monaten, noch besser innerhalb einer Betriebsdauer von wenigstens 30 Monaten, oder von wenigstens 36 Monaten oder mehr ein positives Vorzeichen aufweist.

An dieser Stelle sei festgehalten, dass die Temperaturen der Temperaturzonen A und B ($T^A$ und $T^B$) im großtechnischen Betrieb aus unterschiedlichen Gründen gewissen Schwankungen (in der Regel innerhalb des Intervalls ±20°C bzw. ±10°C liegend) unterliegen können (beispielsweise dann, wenn eine Zwischenregenerierung gemäß der DE-A 10351269 vorgenommen wird; unmittelbar nach erfolgter Zwischenregenerierung (im Vergleich zum Betrieb unmittelbar vor der Zwischenregenerierung) sind in der Regel geringere Temperaturen (in Einzelfällen kann dieser Temperaturunterschied auch bis zu 40°C oder mehr betragen) der Temperaturzonen ausreichend, um unter ansonsten unveränderten Bedingungen den selben, auf den Einmaldurchgang des Reaktionsgasgemischs durch das Katalysatorfestbett bezogenen, Propenumsatz zu gewährleisten). In diesem Fall trägt man den tatsächlichen Verlauf der Temperatur der jeweiligen Zone über die Zeit auf und legt durch die Messpunkte nach der von Legendre und Gauß entwickelten Methode der kleinsten Summe der Abweichungsquadrate eine Ausgleichskurve. Sind auf der Grundlage dieser Ausgleichskurven die erfindungsgemäßen Merkmale erfüllt, wird von der erfindungsgemäßen Verfahrensweise Gebrauch gemacht.

Für den Fall, dass im Verlauf einer erfindungsgemäßen heterogen katalysierten Gasphasen-Partialoxidation von Propen zu Acrolein (dies ist immer auch eine solche von Propen zu Acrolein und Acrylsäure) aufgrund von z.B. veränderter Marktnachfrage oder anderweitig veränderten Rahmenbedingungen im Rahmen des Langzeitbetriebes ein und desselben Katalysatorfestbetts Verfahrensparameter wie z.B. die Propenbelastung des Katalysatorfestbetts, oder die Propenbelastung des Katalysatorfestbetts und der auf einen Einmaldurchgang des Reaktionsgasgemischs durch das Katalysatorfestbett bezogene Propenumsatz mit unmittelbarer Rück-Wirkung (eine solche Veränderung wäre auch eine Erhöhung des Arbeitsdrucks gemäß der DE-A 10 2004 025445) auf die Temperatur der Temperaturzonen A, B verändert werden, um sie so verändert im nachfolgenden weiteren Betrieb über einen längeren Zeitabschnitt (Betriebsabschnitt) im Wesentlichen beizubehalten, so liegt eine erfindungsgemäße Verfahrensweise auch dann vor, wenn in diesem nachfolgenden längeren Betriebsabschnitt mit Bezug auf das Katalysatorfestbett und dessen Betrieb im Wesentlichen "unmittelbar" nach der vorgenannten Veränderung als Betrieb eines "frischen Katalysatorfestbetts" die erfindungsgemäßen kennzeichnenden Merkmale erfüllt werden. Propenbelastungen des Katalysatorfestbetts, die in einem Intervall (X ± 10) Nl/l·h liegen, werden dabei für die erfindungsgemäßen Zwecke als ein und dieselbe Propenbelastung betrachtet. Eine erfindungsgemäße Verfahrensweise liegt auch dann vor, wenn der Langzeitbetrieb bei Propenbelastungen des Katalysatorfestbetts von ≥ 140 Nl Propen/l Katalysatorfestbett h zwischenzeitlich durch einen Niederlastbetrieb (Propenbelastung des Katalysatorfestbetts < 140 Nl/l·h) unterbrochen wird und beim Ausblenden der Niederlastbetriebsphasen ein erfindungsgemäßes Hochlast-Langzeitbetriebsmuster resultiert.

Ferner soll unter dem Verfahren einer heterogen katalysierten partiellen Gasphasenoxidation von Propen zu Acrolein am frisch beschickten Katalysatorfestbett die Ausübung des Verfahrens nach Abschluss einer gegebenenfalls auftretenden Formierung des Katalysatorfestbetts, d.h., nach Erreichen des quasi-stationären Betriebszustandes, verstanden werden.

Generell macht auch derjenige vom erfindungsgemäßen Verfahren Gebrauch, der das erfindungsgemäße Verfahren nur über einen bestimmten Zeitbereich des Langzeitbetriebs betreibt und vorab des teilweisen oder vollständigen Ersatzes des Katalysatorfestbetts durch ein Frischbett die erfindungsgemäße Langzeitbetriebsweise verlässt.

Erfindungsgemäß bevorzugt wird $\Delta T^{BA}$ im Langzeitbetrieb des erfindungsgemäßen Verfahrens 70°C nicht überschreiten. Besonders vorteilhaft wird $\Delta T^{BA}$ im Langzeitbetrieb des erfindungsgemäßen Verfahrens 60°C, und ganz besonders vorteilhaft 50°C nicht überschreiten.

Erfindungsgemäß besonders günstige Verfahren sind jene, bei denen $\Delta T^{BA}$ über die gesamte Betriebsdauer des Langzeitbetriebs im Bereich 10°C bis 50°C, vorzugsweise im Bereich 15°C bis 45°C, und ganz besonders bevorzugt im Bereich 20°C bis 40°C liegt.

Erfindungsgemäße Verfahren sind z.B. solche Verfahren, bei denen sich $\Delta T^{BA}$ mit zunehmender Betriebsdauer um wenigstens 5°C, oder um wenigstens 10°C, oder um wenigstens 15°C, oder um wenigstens 20°C, oder um wenigstens 25°C, oder um wenigstens 30°C erhöht. In der Regel wird sich $\Delta T^{BA}$ beim erfindungsgemäßen Verfahren um nicht mehr als 50°C, meist um nicht mehr als 30°C, häufig um nicht mehr als 20°C erhöhen.

Häufig wird sich beim erfindungsgemäßen Langzeitbetrieb die Temperatur $T^A$ über die gesamte Betriebsdauer im Bereich von 300 bis 400°C, und vorzugsweise im Bereich von 310°C bis 390°C bzw. im Bereich von 320°C bis 380°C bewegen.

Ferner wird sich beim erfindungsgemäßen Langzeitbetrieb die Temperatur $T^B$ über die gesamte Betriebsdauer häufig im Bereich von 305°C bis 415°C, vorzugsweise im Bereich von 315°C bis 410°C und besonders bevorzugt im Bereich von 330°C bis 410°C bewegen.

Bezogen auf einen Einmaldurchgang des Reaktionsgasgemischs durch das Katalysatorfestbett wird der in der Temperaturzone A resultierende Umsatz des Propylens beim erfindungsgemäßen Langzeitbetrieb über die gesamte Betriebsdauer mit Vorteil 50 bis 80 mol-% und mit besonderem Vorteil 55 bis 75 mol-% betragen.

In der Temperaturzone B wird sich der auf einen Einmaldurchgang des Reaktionsgasgemischs bezogene Umsatz des Propens beim erfindungsgemäßen Langzeitbetrieb über die gesamte Betriebsdauer erfindungsgemäß vorteilhaft auf einen Wert ≥ 92 mol-%, oder ≥ 94 mol-%, bevorzugt ≥ 96 mol-% und ganz besonders bevorzugt ≥ 97 mol-%, oder ≥ 98 mol-%, oder ≥ 99 mol-% erhöhen.

In der Regel wird sich der Langzeitbetrieb des erfindungsgemäßen Verfahrens auf wenigstens 2 Betriebsmonate, oder auf wenigstens 4 Betriebsmonate, oder wenigstens 6 Betriebsmonate, bzw. wenigstens 9 Betriebsmonate, oder wenigstens 1 Betriebsjahr, bzw. wenigstens 1,5 Betriebsjahre, oder wenigstens 2 Betriebsjahre, bzw. wenigstens 2,5 Betriebsjahre, bzw. wenigstens 3 Betriebsjahre und teilweise sogar auf wenigstens 5 Betriebsjahre, bzw. wenigstens 7 Betriebsjahre und in Einzelfällen sogar auf 10 Betriebsjahre und mehr erstrecken.

Die über eine Betriebsdauer von 1000 Tagen gemittelte Änderungsrate von $T^B$ kann beim erfindungsgemäßen Verfahren z.B. +0,04°C/Tag betragen, während gleichzeitig die Änderungsrate von $T^A$ über die gleiche Betriebsdauer gemittelt lediglich +0,02°C/Tag beträgt.

In der Regel wird man spätestens dann beginnen, vom erfindungsgemäßen Verfahren Gebrauch zu machen, wenn sich das Katalysatorfestbett in einem solchen Zustand befindet, dass $U^B$ unter ansonsten unveränderten Verfahrensbedingungen um wenigstens 0,2, bzw. wenigstens 0,3, bzw. wenigstens 0,4, oder wenigstens 0,5 Molprozentpunkte niederer läge als der Wert für $U^B$ unter denselben Verfahrensbedingungen am frischen Katalysatorfestbett beträgt.

Die für das erfindungsgemäße Verfahren zu verwendenden Katalysatoren und sonstigen Verfahrensbedingungen wird man im Übrigen anwendungstechnisch zweckmäßig so wählen, dass die Selektivität der Acroleinbildung, bezogen auf das beim Einmaldurchgang des Reaktionsgasgemischs durch das Katalysatorfestbett umgesetzte Propen, vorteilhaft ≥ 82 mol-%, oder ≥ 84 mol-%, oder ≥ 87 mol-%, oder ≥ 89 mol-%, oder ≥ 90 mol-%, oder ≥ 92 mol-%, oder ≥ 94 mol-% beträgt.

Üblicherweise wird bei der erfindungsgemäßen Verfahrensweise als Nebenprodukt Acrylsäure gebildet (bezogen auf die gebildete molare Menge an Acrolein in der Regel in Mengen von ≤ 15 mol-% bzw. ≤ 10 mol-%). Letztere ist insbesondere dann ein gern gesehenes Nebenprodukt, wenn das erfindungsgemäße Verfahren die erste Reaktionsstufe eines zweistufigen Verfahrens der heterogen katalysierten partiellen Gasphasenoxidation von Propen zu Acrylsäure bildet. Fasst man daher Acrolein und Acrylsäure als (gemeinsames) Gesamtzielprodukt zusammen, wird man die für das erfindungsgemäße Verfahren zu verwendenden Katalysatoren und sonstigen Verfahrensbedingungen anwendungstechnisch zweckmäßig so wählen, dass die Selektivität der Gesamtzielproduktbildung, bezogen auf den Propenumsatz beim Einmaldurchgang des Reaktionsgasgemischs durch das Katalysatorfestbett, ≥ 93 mol-%, oder ≥ 95 mol-%, oder

$\geq$ 96 mol-%, oder $\geq$ 97 mol-%, oder $\geq$ 98 mol-% beträgt.

Anwendungstechnisch zweckmäßig erfolgt die Durchführung des erfindungsgemäßen Verfahrens bevorzugt in den bereits angesprochenen Zweizonen-Vielkontaktrohr-Reaktoren. Der radiale Temperaturgradient des Wärmeträgers innerhalb einer Temperaturzone beträgt dabei in der Regel 0,01 bis 5°C, häufig 0,1 bis 2°C, und ist erfindungsgemäß vorteilhaft möglichst gering.

Die Stromstärke des Wärmeträgers wird dabei in der Regel der Lehre der EP-A 700714 folgend so gewählt, dass die Temperatur des Wärmeträgers vom Eintritt in die Temperaturzone bis zum Austritt aus der Temperaturzone (bedingt durch de Exothermie der Reaktion) um 0 bis 15°C ansteigt. In typischer Weise wird das vorgenannte $\Delta T$ erfindungsgemäß 1 bis 10°C, oder 2 bis 8°C, oder 3 bis 6°C betragen. Mit Vorteil ist $\Delta T$ klein.

Grundsätzlich kann das erfindungsgemäße Verfahren jedoch auch in sonstigen zwei Temperaturzonen aufweisenden Reaktoren vom Typ eines indirekten Wärmeaustauschers durchgeführt werden.

Die erfindungsgemäße Verfahrensweise ist gegenüber den im Stand der Technik empfohlenen Verfahrensweisen insbesondere dann vorteilhaft, wenn die Propenbelastung des Katalysatorfestbetts $\geq$ 150 Nl/l·h, oder $\geq$ 160 Nl/l·h, oder $\geq$ 170 Nl/l·h, oder $\geq$ 180 Nl/l·h, oder $\geq$ 190 Nl/l·h, oder $\geq$ 200 Nl/l·h beträgt. In der Regel wird die Propenbelastung des Katalysatorfestbetts beim erfindungsgemäßen Verfahren jedoch $\leq$ 600 Nl/l·h, meist $\leq$ 400 Nl/l·h, oder $\leq$ 300 Nl/l·h, oder $\leq$ 250 Nl/l·h betragen.

Die Vorteilhaftigkeit der erfindungsgemäßen Verfahrensweise mit Blick auf die Selektivität der Acrolein- oder der Acrolein- und Acrylsäurebildung (der Gesamtzielproduktbildung), sowie mit Blick auf den beim Einmaldurchgang des Reaktionsgasgemischs durch das Katalysatorfestbett sich einstellenden Propenumsatz und mit Blick auf die resultierende Standzeit des Katalysatorfestbetts ist vermutlich darin begründet, dass die Exothermie einer heterogen katalysierten partiellen Gasphasenoxidation von Propen zu Acrolein vergleichsweise hoch ist (340 kJ/mol).

Dies führt bei den erfindungsgemäß geforderten Propenbelastungen des Katalysatorfestbetts sowie den auf einen Einmaldurchgang des Reaktionsgasgemischs durch das Katalysatorfestbett geforderten Propenumsätzen innerhalb des Katalysatorfestbetts zur Freisetzung beträchtlicher Reaktionswärmen.

Um auszuschließen, dass das in der Temperaturzone A gebildete Acrolein in der Temperaturzone B zu hohe effektive Festbetttemperaturen durchlaufen muss, welche die unerwünschte Vollverbrennung des Acroleins fördern, muss ein beträchtlicher Anteil des Propenumsatzes zwingend in der Temperaturzone A erfolgen, was in selbiger zur Ausbildung einer ausgeprägten Heißpunkttemperatur führt.

Folgt man nun im Langzeitbetrieb der Lehre der WO 2007/082827 und erhöht über die Betriebsdauer $T^A$ stärker als $T^B$, wie es die WO 2007/082827 einfordert, so werden in der Temperaturzone A offensichtlich wenigstens in Teilbereichen vergleichsweise rasch effektive Katalysatorbetttemperaturen erreicht, die für die Katalysatorqualität prohibitiv sind. Die Katalysatorqualität wird dadurch mit zunehmender Betriebsdauer zunehmend irreversibel gemindert, was darüber hinaus bei weiterer Erhöhung der Katalysatorfestbetttemperatur gleichzeitig eine beträchtliche Erhöhung des Anteils an unerwünschter Vollverbrennung in der Temperaturzone A bedingt. Dies führt schließlich dazu, dass vergleichsweise früh Propenumsatz und/oder Selektivität der Acrolein- bzw. der Acrolein- und Acrylsäurebildung (d.h., die Zielproduktausbeute $Y^{AC}$ bzw. $Y^{AC+AA}$) auch mit der Maßnahme einer zunehmenden Erhöhung der Temperatur des Katalysatorfestbetts nicht mehr aufrecht erhalten werden können, so dass ein Teil- oder Vollwechsel des Katalysatorfestbetts angezeigt ist.

Ein Frühindikator für das baldige Einsetzen des wie ebenda beschriebenen Verhaltens ist häufig der zeitliche Verlauf von $\Delta T^{HB}_A$. Während $\Delta T^{HB}_A$ beim frisch beschickten Katalysatorfestbett mit zunehmender Betriebsdauer und Erhöhung von $T^A$ in aller Regel zunächst zunimmt, setzt bei Anwendung der Verfahrensweise gemäß WO 2007/082827 im Langzeitbetrieb oft vergleichsweise früh der Zeitpunkt ein, ab dem $\Delta T^{HB}_A$ bei Fortsetzung des Betriebs abnimmt. Das Einsetzen dieser Abnahme ist normalerweise ein wesentlicher Fingerzeig für eine bereits ausgeprägte irreversible Schädigung des Katalysatorfestbetts.

Mit Hilfe der erfindungsgemäßen Verfahrensweise kann dem vorstehend beschriebenen Szenario offensichtlich vergleichsweise besser begegnet werden.

D.h., die Bedingung $T^{maxA} - T^{maxB} \geq 0$ allein ist für eine erfolgreiche Langzeitbetriebsweise zwar eine notwendige, aber nicht hinreichende Bedingung. Besonders vorteilhafte Ergebnisse stellen sich vielmehr dann ein, wenn beim Langzeitbetrieb die höchste effektive Temperatur des Katalysatorfestbetts zum Einen möglichst lange in der Temperaturzone A und gleichzeitig bei vergleichsweise niederen Absolutwerten gehalten wird. Letzteres ist bei der erfindungsgemäßen Langzeitbetriebsweise der Fall.

Selbstredend kann die hier beschriebene Verfahrensweise auch bei Propenbelastungen des Katalysatorfestbetts von < 140 Nl/l·h, oder $\leq$ 130 Nl/l·h, oder $\leq$ 120 Nl/l·h, oder $\leq$ 110 Nl/l·h, oder $\leq$ 100 Nl/l·h, oder $\leq$ 90 Nl/l·h, oder $\leq$ 80 Nl/l·h (in der Regel aber $\geq$ 60 Nl/l·h) erfolgreich angewendet werden. Mit zunehmend abnehmender Propenbelastung des Katalysatorfestbetts gewinnt jedoch die Verfahrensweise gemäß der WO 2007/082827 gegenüber einer solchen Verfahrensweise an Vorteilhaftigkeit.

Ebenso kann die hier beschriebene Verfahrensweise analog auch auf eine heterogen katalysierte partielle Gasphasenoxidation von Acrolein zu Acrylsäure angewendet werden. Aufgrund der vergleichsweise geringeren Exothermie dieser Partialoxidation und einer höheren Temperatursensitivität der Totaloxidation (Vollverbrennung) von Acrylsäure im Vergleich zu Acrolein ist in diesem Fall aber auch bei hohen Acroleinbelastungen des Katalysatorfestbetts eine Verfahrensweise gemäß der WO 2007/082827 vorzuziehen.

Nach erfolgter frischer Beschickung des Katalysatorbetts oder nach erfolgter Regenierung des Katalysatorfestbetts (z.B., in dem ein heißes Gasgemisch aus molekularem Sauerstoff und Inertgas durch das Katalysatorfestbett geführt wird, wie es in dem in dieser Schrift diesbezüglich zitierten Stand der Technik beschrieben wird), kann das erfindungsgemäße Verfahren normalerweise nicht unmittelbar bei der für den stationären Betrieb angestrebten hohen Propenbelastung des Katalysatorfestbetts samt zugehörigem, auf einen Einmaldurchgang des Reaktionsgasgemischs durch das Katalysatorfestbett bezogenem, Zielumsatz des Propens aufgenommen werden.

Dies ist dadurch bedingt, dass sich die frisch hergestellten bzw. frisch regenerierten, oxidische Aktivmassen aufweisenden, Katalysatoren in einem quasi hyperaktiven (sauerstoffüberversorgten) Zustand befinden, der bei unmittelbarer Anwendung der stationären Betriebsbedingungen kurzzeitig insbesondere zu extremen Werten für $T^{maxB}$ führen würde.

Diesem Sachverhalt wird in der Praxis normalerweise dadurch begegnet, dass das heterogen katalysierte Partialoxidationsverfahren bei einer solchen Inbetriebnahme bzw. Wiederinbetriebnahme zunächst bei vergleichsweise geringeren Werten für die Propenbelastung des Katalysatorfestbetts und/oder den auf einen Einmaldurchgang des Reaktionsgasgemischs durch das Katalysatorfestbett bezogenen Propenumsatz durchgeführt wird (anwendungstechnisch zweckmäßig wird auch dabei $\Delta T^{BA}$ bei > 0°C gehalten). Anschließend bewegt man sich von diesen Inbetriebnahme-bzw. Wiederinbetriebnahmebedingungen auf die Betriebsbedingungen des angestrebten stationären Betriebs vorteilhaft so zu, dass $T^{maxB}$ stets > $T^{maxB}$ und zu keinem Zeitpunkt $T^{maxB}$ > $T^{maxB}$ im stationären Betriebszustand +3°C (d.h., der $T^{maxA}$-Wert für den stationären Betriebszustand wird auf dem Weg zum stationären Betriebszustand höchstens um 3°C überschritten). Vorteilhaft wird $T^{maxB}$ beim erfindungsgemäßen Verfahren 425°C oder besser 420°C, oder noch besser 410°C nicht überschreiten.

Üblicherweise wird $T^{maxB}$ beim erfindungsgemäßen Verfahren jedoch ≥ 350°C, meist ≥ 360°C und oft ≥ 370°C oder ≥ 380°C betragen.

Die Betriebsphasen des heterogen katalysierten Gasphasenpartialoxidationsverfahrens ab Inbetriebnahme bzw. Wiederinbetriebnahme des Katalysatorfestbetts bis zum Erreichen der Bedingungen des stationären Betriebs (bis zum Erreichen des stationären Betriebs) werden nicht dem erfindungsgemäßen Verfahren des Langzeitbetriebs zugerechnet (in der Regel erstrecken sie sich auf ≤ 72 h, häufig ≤ 48 h bzw. ≤24 h).

Erfindungsgemäß bevorzugt wird man die erfindungsgemäße Verfahrensweise kombiniert mit einer Zwischenregenerierung gemäß der Lehre der DE-A 10351269, WO 2004/085369, DE-A 10350812 oder EP-A 614872 anwenden.

Ferner kann man bevor das Katalysatorfestbett vollständig ausgetauscht wird, einen Teilbettwechsel gemäß der Lehre der DE-A 10232748 bzw. der WO 2004/009525 vornehmen. Dabei kann sich der Teilkatalysatorfestbettwechsel in allen Fällen in Strömungsrichtung des Reaktionsgasgemischs auf bis zu 80%, oder nur auf bis zu 70%, oder nur auf bis zu 60%, oder nur auf bis zu 50%, oder nur auf bis zu 40%, oder nur auf bis zu 30%, oder bevorzugt auf bis zu 25%, besonders bevorzugt auf 30 bis 50% und ganz besonders bevorzugt auf 35 bis 45% der Schüttlänge des jeweiligen Katalysatorfestbetts erstrecken (eine zu 100% aus Inertmaterial bestehende Deckbeschickung (die Erstbeschickung aus der Strömungssicht) wird dabei, wie auch für die sonstigen Belange dieser Schrift, nicht zum Katalysatorfestbett zugehörig betrachtet; in entsprechender Weise wird für die Belange der vorliegenden Erfindung eine zu 100% aus Inertmaterial bestehende Abschlussbeschickung (die Endbeschickung aus der Strömungssicht) normalerweise (so lange nicht explizit etwas anderes gesagt wird) nicht zum Katalysatorfestbett zugehörig betrachtet; eine zu 100% aus Inertmaterial bestehende Zwischenbeschickung wird (so lange nicht explizit etwas anderes gesagt wird) in dieser Schrift jedoch üblicherweise zum Katalysatorfestbett zugehörig betrachtet).

Zweckmäßigerweise beträgt der vorgenannte Prozentsatz für einen Teilkatalystorwechsel häufig nicht weniger als 5, bzw. nicht weniger als 10, bzw. nicht weniger als 20%.

Das molare Verhältnis von $O_2$: $C_3H_6$ im Reaktionsgaseingangsgemisch für eine erfindungsgemäße Partialoxidation von Propen zu Acrolein beträgt erfindungsgemäß ≥ 1. Üblicherweise wird dieses Verhältnis bei Werten ≤ 3 liegen. Häufig beträgt das molare Verhältnis von $O_2$: $C_3H_6$ im Reaktionsgaseingangsgemisch beim erfindungsgemäßen Verfahren ≥ 1,2, bzw. ≥ 1,5 und ≤ 2,0.

Generell ist es für eine erfindungsgemäße heterogen katalysierte Partialoxidation von Propen zu Acrolein günstig, wenn das Produktgasgemisch noch (z.B. bis zu 3 Vol.-%) nicht umgesetzten molekularen Sauerstoff enthält.

Als frische (zuvor noch nicht eingesetzte, noch nicht verwendete) Katalysatoren für das Katalysatorfestbett (die Festbettkatalysatorschüttung) einer erfindungsgemäßen Gasphasen-Partialoxidation von Propen zu Acrolein kommen alle diejenigen in Betracht, deren Aktivmasse wenigstens ein Mo, Bi und Fe enthaltendes Multimetalloxid ist.

Erfindungsgemäß vorteilhafte Katalysatoren sind jene, deren Aktivmasse ein Multimetalloxid ist, das wenigstens die Elemente Mo, Fe und Bi, sowie zusätzlich wenigstens eines der beiden Elemente Ni und Co enthält. Dabei erweist es sich als günstig, wenn von den vorgenannten fünf (von Sauerstoff verschiedenen) Elementen, bezogen auf ihre in der Aktivmasse enthaltene molare Gesamtmenge G, auf das Element Mo der größte molare Anteil (in mol-% bezogen auf die molare Gesamtmenge G) entfällt.

Erfindungsgemäß besonders geeignete Multielementoxidaktivmassen sind somit insbesondere jene der allgemeinen Formel I der DE-A 19955176, die Multielementoxidaktivmassen der allgemeinen Formel I der DE-A 19948523, die Multielementoxidaktivmassen der allgemeinen Formel I, II und III der DE-A 10101695, die Multielementoxidaktivmassen der allgemeinen Formeln I, II und III der DE-A 19948248 und die Multielementoxidaktivmassen der allgemeinen Formeln I, II und III der DE-A 19955168 sowie die in den Schriften EP-A 700 714, DE-A 102009047291, DE-A 102007005606, DE-A 10200840094, WO 2008/087116, WO 2008/087115, WO 2007/017431, DE-A 10 2007 004961, DE-A-10 2008 040093, DE-Anmeldenummer 102008042064.6, DE-Anmeldenummer 102008042061.1 und DE-A 102008042060 genannten Multielementoxidaktivmassen. Dies gilt insbesondere für die in den fünf letztgenannten Schriften offenbarten beispielhaften Ausführungsformen. Ebenso eignen sich für das erfindungsgemäße Verfahren als Multielementoxidaktivmassen diejenigen, die in der WO 2007/082827 und in der WO 2005/042459 für die heterogen katalysierte partielle Gasphasenpartialoxidation empfohlen werden.

Ferner eignen sich für die frische Festbettkatalysatorschüttung einer erfindungsgemäßen Propenpartialoxidation die Mo, Bi und Fe enthaltenden Multimetalloxidkatalysatoren, die in den Schriften Research Disclosure Nr. 497012 vom 29.08.2005, DE-A 100 46 957, DE-A 100 63 162, DE-C 33 38 380, DE-A 199 02 562, EP-A 15 565, DE-C 23 80 765, EP-A 807 465, EP-A 279 374, DE-A 33 00 044, EP-A 575 897, US-A 4,438,217, DE-A 198 55 913, WO 98/24746, DE-A 197 46 210 (diejenigen der allgemeinen Formel II), JP-A 91/294239 und EP-A 293 224 offenbart werden. Dies gilt insbesondere für die beispielhaften (einschließlich der Vergleichsbeispiele) Ausführungsformen in diesen Schriften, unter denen jene der Research Disclosure Nr. 497012, der EP-A 15 565, der EP-A 575 897, der DE-A 197 46 210 und der DE-A 198 55 913 besonders bevorzugt werden. Besonders hervorzuheben sind in diesem Zusammenhang ein Katalysator gemäß Beispiel 1c aus der EP-A 15 565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}Ox \cdot 10\ SiO_2$ aufweist. Ferner sind hervorzuheben das Beispiel mit der laufenden Nr. 3 aus der DE-A 198 55 913 (Stöchiometrie: $Mo_{12}Co_7Fe_3Bi_{0,6}K_{0,08}Si_{1,6}Ox$) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 101 01 695 bzw. WO 02/062737.

Weiterhin sind das Beispiel 1 aus der DE-A 100 46 957 (Stöchiometrie: $[Bi_2W_2O_9 \times 2WO_3]_{0,5} \cdot [Mo_{12}Co_{5,6}Fe_{2,94}Si_{1,59}K_{0,08}Ox]_1$) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 100 63 162 (Stöchiometrie: $Mo_{12}Bi_{1,0}Fe_3Co_7Si_{1,6}K_{0,08}$), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für das erfindungsgemäße Verfahren geeigneten Multielementoxidaktivmassen lässt sich unter der allgemeinen Formel I,

$$Mo_{12}Bi_aFe_bX^1_cX^2_dX^3_eX^4_fO_n \qquad (I),$$

in der die Variablen nachfolgende Bedeutung aufweisen:

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,
$a$ = 0,05 bis 5,
$b$ = 0,01 bis 5, vorzugsweise 2 bis 4,
$c$ = 0,1 bis 10, vorzugsweise 3 bis 10,
$d$ = 0 bis 2, vorzugsweise 0,02 bis 2,
$e$ = 0 bis 8, vorzugsweise 0 bis 5,
$f$ = 0 bis 10 und
$n$ = eine Zahl, die durch die Wertigkeit und Häufigkeit der von Sauerstoff verschiedenen Elemente in I bestimmt wird,

subsumieren.

Sie sind in an sich bekannter Weise erhältlich (siehe z.B. die DE-A 40 23 239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C kalciniert. Die Kalcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Kalcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Kalcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Kalcination unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Üblicherweise werden die Multimetalloxidaktivmassen der allgemeinen Formel I in der (frischen) Festbettkatalysatorschüttung für eine erfindungsgemäße Gasphasenpartialoxidation zu Acrolein nicht in Pulverform sondern zu bestimmten Katalysatorgeometrien geformt eingesetzt, wobei die Formgebung vor oder nach der abschließenden Kalcination erfolgen kann. Beispielsweise können aus der Pulverform der Aktivmasse oder ihrer unkalcinierten und/oder partiell kalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formgebungshilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell kalcinierten Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der erfindungsgemäßen Zielreaktion in der Regel im Wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im Wesentlichen unporösen, oberflächenrauhen, kugelförmigen Trägern aus Steatit (z.B. Steatit C 220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die (frischen) Katalysatoren einer erfindungsgemäßen Partialoxidation zur Herstellung von Acrolein geeignete Multimetalloxidaktivmassen sind ferner Massen der allgemeinen Formel II,

$$[Y^1{}_{a'}Y^2{}_{b'}O_{x'}]_p[Y^3{}_{c'}Y^4{}_{d'}Y^5{}_{e'}Y^6{}_{f'}Y^7{}_{g'}Y^8{}_{h'}O_{y'}]_q \qquad (II),$$

in der die Variablen folgende Bedeutung haben:

$Y^1$ = nur Wismut oder Wismut und wenigstens eines der Elemente Tellur, Antimon, Zinn und Kupfer,
$Y^2$ = Molybdän, oder Wolfram, oder Molybdän und Wolfram,
$Y^3$ = ein Alkalimetall, Thallium und/oder Samarium,
$Y^4$ = Nickel und/oder Kobalt, sowie gegebenenfalls eines oder mehrere der Elemente Kupfer, Mangan, Zink, Zinn, Cadmium, Quecksilber und die Erdalkalimetalle,
$Y^5$ = Eisen oder Eisen und wenigstens eines der Elemente Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
$Y^8$ = Molybdän, oder Molybdän und Wolfram,
a' = 0,01 bis 8,
b' = 0,1 bis 30,
c' = 0 bis 4,
d' = 0,1 bis 20,
e' > 0 bis 20, vorzugsweise 0,01 oder 0,1 bis 20,
f' = 0 bis 6,
g' = 0 bis 15,
h' = 8 bis 16,
x',y' = Zahlen, die durch die Wertigkeit und Häufigkeit der von Sauerstoff verschiedenen Elemente in II bestimmt werden und
p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte Bereiche der chemischen Zusammensetzung $Y^1{}_{a'}Y^2{}_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. bis 25 $\mu$m, beträgt.

Besonders vorteilhafte erfindungsgemäße Multimetalloxidmassen II sind solche, in denen $Y^1$ nur Wismut ist.

Unter diesen werden wiederum jene bevorzugt, die der allgemeinen Formel III,

$$[Bi_{a''}Z^2{}_{b''}O_{x''}]_{p''} [Z^8{}_{12}Z^3{}_{c''}\text{-}Z^4{}_{d''}Fe_{e''}Z^5{}_{f''}Z^6{}_{g''}Z^7{}_{h''}O_{y''}]_{q''} \qquad (III),$$

in der die Varianten folgende Bedeutung haben:

$Z^2$ = Molybdän, oder Wolfram, oder Molybdän und Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer und/oder Blei,
$Z^6$ = Silicium, Aluminium, Titan und/oder Zirkonium,
$Z^7$ = Kupfer, Silber und/oder Gold,
$Z^8$ = Molybdän, oder Molybdän und Wolfram,
a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,

x",y"= Zahlen, die durch die Wertigkeit und Häufigkeit der von Sauerstoff verschiedenen Element in III bestimmt werden,

p",q"= Zahlen, deren Verhältnis p"/q" 0,1 bis 5, vorzugsweise 0,5 bis 2 beträgt,

entsprechen, wobei diejenigen Massen III ganz besonders bevorzugt werden, in denen $Z^2_{b}" = (Wolfram)_{b"}$ und $Z^8_{12} = (Molybdän)_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils $[Y^1_{a'}Y^2_{b'}O_{x'}]_p$ $([Bi_{a"}Z^2_{b"}O_{x'}]_{p"})$ der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}[Bi_{a"}Z^2_{b"}O_{x"}]$ vorliegen, deren Größtdurchmesser im Bereich 1 nm bis 100 $\mu$m liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der DE Anmeldenummer 102008054586.4, DE-A 102008040093, DE-A 102008040094, EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung des entsprechenden Katalysatorfestbetts, bzw. als deren schützende Vorschüttung bzw. als Nachschüttung in Betracht.

Prinzipiell kann bei einer erfindungsgemäßen Partialoxidation von Propen zu Acrolein die volumenspezifische Aktivität des (frischen) Katalysatorfestbetts in Strömungsrichtung des Reaktionsgasgemischs über die Länge des Strömungsweges (d.h., über die Länge des Katalysatorfestbetts) konstant sein, oder vorteilhaft wenigstens einmal (kontinuierlich, oder abrupt oder stufenförmig) zunehmen. Dabei ist es von Vorteil, wenn sich die Aktivmassenzusammensetzung über die Länge des Strömungsweges des Reaktionsgasgemischs (d.h. innerhalb des frischen Katalysatorfestbetts) nicht ändert.

Erfindungsgemäß vorteilhaft ist es, wenn die Festbettkatalysatorschüttung aus wenigstens zwei räumlich aufeinanderfolgenden Festbettkatalysatorschüttungszonen besteht, wobei die volumenspezifische Aktivität innerhalb einer Festbettkatalysatorschüttungszone im Wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt.

Die volumenspezifische (d.h., die auf die Einheit des jeweiligen Schüttungsvolumens normierte) Aktivität einer Festbettkatalysatorschüttungszone kann nun in über die Festbettkatalysatorschüttungszone im Wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im Wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eigenen.

Als solche Materialien kommen z.B. poröse oder unporöse Aluminiumoxide, Siliciumdioxid, Thoriumdioxid, Zirkondioxid, Siliziumcarbid, Silikate wie Magnesium- oder Aluminiumsilikat oder der bereits erwähnte Steatit (z.B. Steatit C 220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wie bereits gesagt, wenn sich die chemische Zusammensetzung der verwendeten Aktivmasse über die gesamte Festbettkatalysatorschüttung nicht verändert. D.h., die für einen einzelnen Katalysatorformkörper verwendete Aktivmasse kann zwar ein Gemisch aus verschiedenen, z.B. die Elemente Mo, Fe und Bi enthaltenden, Multimetalloxiden sein, für alle Katalysatorformkörper der Festbettkatalysatorschüttung ist dann jedoch das gleiche Gemisch zu verwenden.

Eine in Strömungsrichtung des Reaktionsgasgemisches über die Festbettkatalysatorschüttung zonenweise zunehmende (ist besonders vorteilhaft) volumenspezifische Aktivität lässt sich somit in einfacher Weise z.B. dadurch einstellen, dass man die Schüttung in einer ersten Festbettkatalysatorschüttungszone mit einem hohen Anteil an inerten Verdünnungsverformkörpern bezogen auf eine Sorte von Katalysatorformkörpern beginnt, und dann diesen Anteil an Verdünnungsformkörpern in Strömungsrichtung zonenweise verringert.

Eine solche erfindungsgemäß vorteilhafte zonenweise Zunahme der volumenspezifischen Aktivität ist aber auch z.B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassenanteil steigert. Alternativ kann man auch die Aktivmassen selbst verdünnen, in dem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z.B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden, so dass die volumenspezifische Aktivität in Strömungsrichtung des Reaktionsgasgemischs zonenweise zunimmt.

Vorab und/oder im Anschluss an die Festbettkatalysatorschüttung können sich ausschließlich aus Inertmaterial (z.B. nur Verdünnungsformkörpern) bestehende Schüttungen befinden (sie werden in dieser Schrift begrifflich nicht der Festbettkatalysatorschüttung zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstelle ringförmig).

Häufig weisen die für solche Inertschüttungen verwendeten Formkörper die ringförmige Geometrie 7 mm x 7 mm x 4 mm bzw. 7 mm x 3 mm x 4 mm (jeweils Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die Inertschüttungen erstrecken. Erfindungsgemäß vorteilhaft erfassen sowohl die Temperaturzone A als auch die Temperaturzone B für eine erfindungsgemäße Partialoxidation von Propen zu Acrolein jeweils nicht mehr als drei Festbettkatalysatorschüttungszonen (erfindungsgemäß vorteilhaft wird wenigstens eine Festbettkatalysatorschüttungszone von beiden Temperaturzonen erfasst).

Erfindungsgemäß besonders bevorzugt umfasst die gesamte Festbettkatalysatorschüttung nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches) der Festbettkatalysatorschüttung sollte (bei einheitlicher Aktivmasse über die gesamte Festbettkatalysatorschüttung) die volumenspezifische Aktivmasse (d.h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung). In der Regel wird diese Zunahme beim erfindungsgemäßen Verfahren einer heterogen katalysierten Partialoxidation von Propen zu Acrolein nicht mehr als 50 Gew.-%, meist nicht mehr als 40 Gew.-% betragen. Ferner sollte bei einheitlicher Aktivmasse über die gesamte Festbettkatalysatorschüttung der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß vorteilhaft nicht mehr als 50 Gew.-%, vorzugsweise nicht mehr als 40 Gew.-% und in der Regel nicht mehr als 30 Gew.-% betragen.

Häufig wird bei einem erfindungsgemäßen Verfahren der heterogen katalysierten Partialoxidation von Propen zu Acrolein die Festbettkatalysatorschüttung aus nur zwei Festbettkatalysatorschüttungszonen bestehen.

Erfindungsgemäß bevorzugt ist die in Strömungsrichtung des Reaktionsgasgemisches letzte Festbettkatalysatorschüttungszone der Festbettkatalysatorschüttung unverdünnt. D.h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z.B. durch Verdünnung mit Inertmaterial abgesenkt, z.B. um 10 %, ist

Besteht die Festbettkatalysatorschüttung für eine heterogen katalysierte Partialoxidation von Propen zu Acrolein nur aus zwei Festbettkatalysatorschüttungszonen, ist es erfindungsgemäß in der Regel vorteilhaft, wenn die Festbettkatalysatorschüttungszone mit der höchsten volumenspezifischen Aktivität nicht bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom (über den Reaktor betrachtet) zum Reaktionsgasgemisch strömt). D.h., in günstiger Weise wird die Festbettkatalysatorschüttungszone mit der geringeren volumenspezifischen Aktivität in die Temperaturzone B hineinragen und die Festbettkatalysatorschüttungszone mit der höheren volumenspezifischen Aktivität in der Temperaturzone B beginnen und enden (d.h., hinter dem Übergang von der Temperaturzone A in die Temperaturzone B ihren Anfang haben).

Insbesondere bei Mitverwendung von, bezogen auf das Reaktionsgaseingangsgemisch, z.B. bis zu 50 Vol.-% Propan als inertem Verdünnungsgas, hat es sich jedoch als zweckmäßig erwiesen, die Festbettkatalysatorschüttungszone mit der höchsten volumenspezifischen Aktivität in die Temperaturzone A hineinragen zu lassen. Dies insbesondere bei Gegenstromfahrweise (über den Reaktor betrachtet) von Salzbädern und Reaktionsgasgemisch.

Besteht die Festbettkatalysatorschüttung nur aus drei Festbettkatalysatorschüttungszonen, ist es erfindungsgemäß in der Regel gleichfalls vorteilhaft, wenn die Festbettkatalysatorschüttungszone mit der höheren volumenspezifischen Aktivität nicht bis in die Temperaturzone A hineinragt sondern in der Temperaturzone B beginnt und endet, d.h., hinter dem Übergang von der Temperaturzone A in die Temperaturzone B ihren Anfang hat (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D.h., normalerweise wird in diesem Fall die Festbettkatalysatorschüttungszone mit der zweithöchsten volumenspezifischen Aktivität sowohl in die Temperaturzone A als auch in die Temperaturzone B hineinragen.

Besteht die Festbettkatalysatorschüttung aus vier Festbettkatalysatorschüttungszonen, ist es erfindungsgemäß in der Regel vorteilhaft, wenn die Festbettkatalysatorschüttungszone mit der dritthöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt).

Im Fall einer Gleichstromführung von Reaktionsgasgemisch und Wärmeträgern in den Temperaturzonen A und B kann es vorteilhaft sein, wenn beim erfindungsgemäßen Verfahren innerhalb der Festbettkatalysatorschüttung die Festbettkatalysatorschüttungszone mit der höchstens volumenspezifischen Aktivität in die Temperaturzone A hineinragt.

Generell lässt sich die volumenspezifische Aktivität zwischen zwei Festbettkatalysatorschüttungszonen einer Festbettkatalysatorschüttung experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleiche Propen enthaltende Reaktionsgasgemisch geführt wird. Die höhere umgesetzte Menge an Propen weist die höhere volumenspezifische Aktivität aus.

Beträgt die Gesamtlänge der Festbettkatalysatorschüttung L, ist es erfindungsgemäß vorteilhaft, wenn sich im Bereich $X \pm L\, \dfrac{4}{100}$, bzw. im Bereich $X \pm L\, \dfrac{3}{100}$, bzw. im Bereich $X \pm L\, \dfrac{2}{100}$ kein Übergang von einer Festbettkatalysatorschüttungszone in eine andere Festbettkatalysatorschüttungszone befindet, wobei X der Ort (die Stelle) innerhalb der Festbettkatalysatorschüttung ist, an dem der Übergang von der Temperaturzone A in die Temperaturzone B erfolgt.

Erfindungsgemäß bevorzugt ist beim erfindungsgemäßen Verfahren die Festbettkatalysatorschüttung (ohne Einbezug von bloßen inerten Vor- und/oder Nachschüttungen) in Strömungsrichtung des Reaktionsgasgemisches wie folgt strukturiert.

Zunächst auf einer Länge von 10 bis 60 %, bevorzugt 20 bis 55 %, besonders bevorzugt 30 bis 55 % und ganz besonders bevorzugt 40 bis 50 % (d.h. z.B. auf einer Länge von 0,90 bis 1,70 m, bevorzugt 1,10 bis 1,60 m), jeweils der Gesamtlänge der Festbettkatalysatorschüttung, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung befindet sich dann erfindungsgemäß vorteilhaft bis zum Ende der Länge der Festbettkatalysatorschüttung (d.h., z.B. auf einer Länge von 1,00 (bzw. 1,40 m) bis 3,00 m, bevorzugt 1,00 bis 2,50 bzw. bis 2,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das Vorgenannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der am Ende der Festbettkatalysatorschüttung gegebenenfalls verwendeten Schalenkatalysatorformkörper.

Eine reine Inertmaterialschüttung, deren Länge, bezogen auf die Länge der Festbettkatalysatorschüttung, zweckmäßig 5 bis 20 % beträgt, führt in Strömungsrichtung des Reaktionsgasgemisches in der Regel auf die Festbettkatalysatorschüttung hin. Sie wird normalerweise als Aufheizzone für das Reaktionsgasgemisch genutzt.

Erfindungsgemäß vorteilhaft erstreckt sich nun bei den vorgenannten Festbettkatalysatorschüttungen die Festbettkatalysatorschüttungszone mit der geringeren volumenspezifischen Aktivität noch auf 5 bis 20 %, häufig auf 5 bis 15 % ihrer Länge in die Temperaturzone B.

Erfindungsgemäß zweckmäßig erstreckt sich die Temperaturzone A auch auf eine zur Festbettkatalysatorschüttung gegebenenfalls angewandte Vorschüttung aus Inertmaterial.

Selbstredend können sich beim erfindungsgemäßen Verfahren an die Temperaturzonen A, B weitere zusätzliche Temperaturzonen anschließen. Dies ist erfindungsgemäß jedoch nicht bevorzugt.

Für Schalenkatalysatoren der Festbettkatalysatorschüttung eignen sich insbesondere Trägerkörper, die eine erhöhte Oberflächenrauhigkeit aufweisen, da diese in der Regel eine erhöhte Haftfestigkeit der aufgebrachten Schale an Aktivmasse bedingt.

Vorzugsweise liegt die Oberflächenrauhigkeit Rz des Trägerkörpers im Bereich von 30 bis 200 $\mu$m, vorzugsweise 30 bis 100 $\mu$m (bestimmt gemäß DIN 4768 Blatt 1 mit einem "Hommel Tester" für DIN-ISO Oberflächenmessgrößen der Fa. Hommelwerke). Das Vorgenannte gilt insbesondere für Trägerkörper aus Steatit C 220 der Fa. CeramTec.. Grundsätzlich können die Trägermaterialien porös oder unporös sein.

In anwendungstechnisch zweckmäßiger Weise erfolgt die Durchführung eines erfindungsgemäßen Verfahrens der Partialoxidation von Propen zu Acrolein in einem Zweizonenrohrbündelreaktor, wie er z.B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224, der WO 2007/082827, der WO 2004/08362, der EP-A 1547994 und der DE-A 2903218 offenbarten Zweizonenrohrbündelreaktoren sind für die Durchführung eines solchen Verfahrens geeignet.

D.h., in einfachster Weise befindet sich die für ein solches Verfahren erfindungsgemäß zu verwendende Festbettkatalysatorschüttung (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D.h., in einfachster Weise umströmt z.B. ein Salzbad A denjenigen Abschnitt der Rohre (die Temperaturzone A), in welchem sich die oxidative Umsetzung des Propens (beim einfachen Durchgang) bis zum Erreichen eines Umsatzes $U^A$ im erfindungsgemäß erforderlichen Bereich vollzieht und ein Salzbad B umströmt den Abschnitt der Rohre (die Temperaturzone B), in welchem sich die oxidative Anschlussumsetzung des Propens (beim einfachen Durchgang) bis zum Erreichen eines Umsatzwertes $U^B$ von wenigstens 90 mol-% vollzieht (bei Bedarf können sich an die erfindungsgemäß anzuwendenden Temperaturzonen A, B weitere Temperaturzonen anschließen, die auf individuellen Temperaturen gehalten werden).

Anwendungstechnisch zweckmäßig umfasst eine erfindungsgemäße Propenpartialoxidation zu Acrolein keine solchen weiteren Temperaturzonen. D.h., das Salzbad B umströmt zweckmäßig den Abschnitt der Rohre, in welchem sich die oxidative Anschlussumsetzung des Propens (beim einfachen Durchgang) bis zu einem Umsatzwert $\geq$ 90 mol-%, oder $\geq$ 92 mol-% oder $\geq$ 94 mol-% oder mehr vollzieht.

Die beiden Salzbäder A, B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstverständlich kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700714, oder in der EP-A 700893, oder in der EP-A 1547944 beschriebenen Rohrbündelreaktor entspricht und insgesamt im Längsschnitt durch das Kontaktrohrbündel ein mäanderförmiger Strömungsverlauf des Wärmeaustauschmittels resultiert.

Die Fließgeschwindigkeit des fluiden Wärmeträgers wird dabei gemäß der Lehre der vorgenannten Schriften vorteilhaft so bemessen, dass bei einer darüber hinausgehenden Erhöhung dieser Fließgeschwindigkeit im Wesentlichen keine Erhöhung des Wärmedurchgangs aus dem Reaktionsrohrinneren auf den Wärmeträger bewirkt wird.

D.h., typische Wärmeübergangszahlen von der materiellen Einhüllenden des Reaktionsraums (z.B. von der Außenwand des Reaktionsrohrs) auf den fluiden Wärmeträger (z.B. auf die Salzschmelze) betragen beim erfindungsgemäßen Verfahren vorteilhaft $\geq$ 700 W/m$^2 \cdot$K, besonders vorteilhaft $\geq$ 1000 W/m$^2 \cdot$K, ganz besonders vorteilhaft $\geq$ 1500 oder $\geq$ 2000 W/m$^2 \cdot$K, in der Regel jedoch $\leq$ 3000 W/m$^2 \cdot$K.

Zweckmäßigerweise wird ein Reaktionsgaseingangsgemisch der Festbettkatalysatorschüttung auf die Temperatur der Temperaturzone A vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder bis 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel ist bei allen vorstehend erwähnten Konstellationen der Stromführung in den Zweizonenrohrbündelreaktoren die Fließgeschwindigkeit der Wärmeaustauschmittels innerhalb der beiden erforderlichen Wärmeaustauschmittelkreisläufen so bemessen, dass die Temperatur des Wärmeaustauschmittels von der Eintrittsstelle in die Temperaturzone bis zur Austrittstelle aus der Temperaturzone (bedingt durch die Exothermie der Reaktion) um 0 bis 15°C ansteigt. D.h., das vorgenannte $\Delta T$ kann erfindungsgemäß 1 bis 10°C, oder 2 bis 8°C oder 3 bis 6°C betragen. Erfindungsgemäß bevorzugt ist es gering.

Die Eintrittstemperaturen der Wärmeaustauschmittel in die Temperaturzonen A, B der Zweizonenrohrbündelreaktoren sind dabei bei einer Propenpartialoxidation zu Acrolein erfindungsgemäß so zu wählen, dass sie den für diese Reaktion in dieser Schrift für die Temperaturzonen A, B geforderten Temperaturen sowie Temperaturunterschieden $\Delta T^{BA}$ entsprechen (bzw. diese bedingen). Im erfindungsgemäßen Langzeitbetrieb sind sie erfindungsgemäß zu verändern.

Es sei an dieser Stelle auch noch einmal darauf hingewiesen, dass für die Durchführung einer erfindungsgemäßen Propenpartialoxidation zu Acrolein insbesondere auch der in der DE-AS 2201528 beschriebene Zweizonenrohrbündelreaktortyp verwendet werden kann, der die Möglichkeit beinhaltet, vom heißen Wärmeaustauschmittel der Temperaturzone B eine Teilmenge an die Temperaturzone A abzuführen, um gegebenenfalls ein Anwärmen eines kalten Reaktionsgaseingangsgemisches oder eines kalten Kreisgases zu bewirken. Ferner kann die Rohrbündelcharakteristik innerhalb einer individuellen Temperaturzone wie in der EP-A 382098 beschrieben gestaltet werden.

Im Fall einer zweistufigen heterogen katalysierten Partialoxidation von Propen zu Acrylsäure wird man hinter der erfindungsgemäßen Verfahrensstufe anwendungstechnisch zweckmäßig eine zweite Zweizonenverfahrensstufe für die heterogen katalysierte partielle Gasphasenoxidation des Acroleins zur Acrylsäure schalten und so betreiben, wie es in der WO 2007/082827 beschrieben ist.

Dabei können selbstredend zwei hintereinander geschaltete Zweizonenrohrbündelreaktoren zu einem Vierzonenrohrbündelreaktor verschmolzen werden, wie es die WO 01/36364 beschreibt (vgl. auch WO 2007/082827, Seite 51).

Zur experimentellen Bestimmung der effektiven Temperatur des Katalysatorfestbetts in einem Reaktionsrohr eines Rohrbündelreaktors kann dieses eine zentriert durch das Reaktionsrohr von oben nach unten verlaufende Thermohülse aufweisen, in welcher mit Hilfe von in der Thermohülse geführten Thermoelementen die effektive Katalysatorfestbetttemperatur (die Reaktionstemperatur) über die gesamte Reaktionsrohrlänge ermittelt werden kann. Grundsätzlich könnte jedes in einem Rohrbündelreaktor befindliche und mit dem Katalysatorfestbett beschickte Reaktionsrohr wie vorstehend beschrieben ausgerüstet sein.

Anwendungstechnisch zweckmäßig weist ein Rohrbündelreaktor jedoch nur eine begrenzte Anzahl derartiger Thermoreaktionsrohre oder auch kurz nur "Thermorohre" auf (vgl. z.B. Seite 56 der WO 2007/082827, die EP-A 873783, die EP-A 1270065 und die US 7,534,339 B2).

Da Thermorohre zusätzlich zum Katalysatorfestbett noch die Thermohülse aufnehmen müssen, würden sie bei ansonsten identischer Rohrgestaltung zwar eine gleich große Wärmeaustauschoberfläche, aber einen geringeren freien, vom Katalysatorfestbett einnehmbaren, Querschnitt als ein bloßes "Reaktionsrohr" aufweisen. Dem wird dadurch Rechnung getragen, dass man sie (die Thermorohre) so gestaltet, dass das Verhältnis von freier Querschnittsfläche im Rohr zum Umfang des Rohres bei Thermorohr und Reaktionsrohr gleich sind. Im Übrigen weisen Reaktionsrohr und Thermorohr bei identischer Rohrlänge über ihre Rohrlänge jeweils dieselbe Katalysatorfestbettstruktur auf. Bei der Beschickung mit Katalysatorfestbett ist darüber hinaus darauf zu achten, dass das Profil des bei der Durchströmung von Reaktionsrohr bzw. Thermorohr mit Reaktionsgasgemisch sich über die Rohrlänge jeweils einstellende Druckverlustprofils in beiden Rohrtypen einheitlich gestaltet wird. Über die Geschwindigkeit der Befüllung der Rohre mit den Formkörpern und/oder durch Mitverwendung von zerkleinerten (versplitteten Formkörpern) kann in entsprechender Weise Einfluss genommen werden (vgl. z.B. EP-A 873783 und US 7,534,339 B2). Insgesamt wird auf diese Weise sichergestellt, dass ein Thermorohr und ein Reaktionsrohr entlang der gesamten Rohrlänge zueinander gleiche Verhältnisse von Reaktionswärmeentwicklung im Rohrinneren und Abfuhr von Reaktionswärme aus dem Rohrinneren aufweisen. Das Thermorohr vermag so repräsentativ für viele Reaktionsrohre den Verlauf der effektiven Katalysatorfestbetttemperatur im Reaktionsrohr abzubilden.

Generell ist es günstig, eine erfindungsgemäße Partialoxidation von Propen zu Acrolein so zu betreiben, dass der Propengehalt im Produktgasgemisch dieser Partialoxidation den Wert 10000 Gew.-ppm, vorzugsweise 6000 Gew.-ppm und besonders bevorzugt 4000 bis 2000 Gew.-ppm nicht übersteigt.

Der Propenanteil im Reaktionsgaseingangsgemisch kann beim erfindungsgemäßen Verfahren z.B. bei Werten von 2 bis 25 Vol.-%, oft 3 bis 20 Vol.-%, oder 4 bis 15 Vol.-%, häufig bei 5 bis 12 Vol.-% bzw. 6 bis 8 Vol.-% liegen (jeweils bezogen auf das Gesamtvolumen). Als Propenquelle eignen sich insbesondere "polymer grade Propen" und "chemical grade Propen" gemäß WO 2004/009525.

Häufig wird man das erfindungsgemäße Propen -> Acrolein Verfahren bei einem Propen : Sauerstoff : indifferente Gase (einschließlich Wasserdampf) Volumenverhältnis im Reaktionsgasausgangsgemisch von 1 : (1,0 bis 3,0) : (5 bis 25), vorzugsweise 1 : (1,7 bis 2,3) : (10 bis 15) durchführen. Im Regelfall wird das indifferente (inerte) Gas zu wenigstens 20 % seines Volumens aus molekularem Stickstoff bestehen. Es kann aber auch zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zur $\geq$ 80 Vol.-%, oder zu $\geq$ 90 Vol.-%, oder zu $\geq$ 95 Vol.-% aus molekularem Stickstoff bestehen (mögliche Inertgase sind neben molekularem Stickstoff z.B. Gase wie Propan, Ethan, Methan, Pentan, Butan, $CO_2$, CO, Wasserdampf und/oder Edelgase). Natürlich kann das inerte Verdünnungsgas bei einer erfindungsgemäßen Propenpartialoxidation zu Acrolein auch zu bis zu 50 mol-%, bzw. bis zu 75 mol-% und mehr aus Propan bestehen (beispielsweise kommen für das erfindungsgemäße Verfahren alle in den Schriften WO 2007/13504, WO 2007/060036, WO 2007/042457, WO 2006/002713, WO 2006/002708 und WO 2006/002703 für die Propenpartialoxidation zu Acrolein offenbarten Reaktionsgaseingangsgemische in Betracht). Bestandteil des Verdünnungsgases kann auch Kreisgas sein, wie es bei der zweistufigen Propenpartialoxidation zu Acrylsäure nach der Abtrennung der Acrylsäure aus dem Produktgasgemisch verbleibt.

Vorgenannte Zusammensetzungsbereiche gelten auch für solche zweistufigen Verfahren, und zwar sowohl in Fällen von Sekundärgaszufuhr (z.B. Zufuhr von Luft und/oder Inertgas zwischen erster und zweiter Reaktionsstufe) als auch in Fällen wo kein Sekundärgas zugeführt wird.

Erfindungsgemäß geeignete Reaktionsgaseingangsgemische sind z.B. solche, die aus

| | |
|---|---|
| 5 bis 15 (bevorzugt 6 bis 11) Vol-% | Propen, |
| 0,5 oder 4 bis 20 (bevorzugt 6 bis 12) Vol.-% | Wasser, |
| $\geq$ 0 bis 10 (bevorzugt $\geq$ 0 bis 5) Vol.-% | von Propen, Wasser, molekularem Sauerstoff und molekularem Stickstoff verschiedenen Bestandteilen, |

soviel molekularem Sauerstoff, dass das molare Verhältnis von im Reaktionsgaseingangsgemisch enthaltenem molekularem Sauerstoff zu darin enthaltenem Propen 1,5 bis 2,5 (bevorzugt 1,6 bis 2,2) beträgt, und als Restmenge bis zu 100 Vol.-% Gesamtmenge aus molekularem Stickstoff zusammengesetzt sind, wie sie die DE-A 10302715 empfiehlt.

Insbesondere bei besonders hohen Propenbelastungen der Festbettkatalysatorschüttung wird die Mitverwendung von inerten Verdünnungsgasen mit hoher spezifischer Wärme empfohlen.

Insbesondere im Fall einer zweistufigen heterogen katalysierten Gas-Phasen-Partialoxidation von Propen zu Acrylsäure, bei der das erfindungsgemäße Verfahren lediglich die erste Reaktionsstufe bildet, kommen auch die nachfolgenden Beschaffenheiten des Reaktionsgaseingangsgemisch in Betracht.

Beispielsweise kann das Reaktionsgaseingangsgemisch $\geq$ 0,01, oder $\geq$ 0,1, oder $\geq$ 0,5, oder $\geq$ 2 Vol.-% an $CO_2$ enthalten. Meist wird der vorgenannten $CO_2$-Gehalt $\leq$ 25 Vol.-% betragen.

Insbesondere dann, wenn als Quelle für den molekularen Sauerstoff beim erfindungsgemäßen Verfahren Luft verwendet wird, wird das Reaktionsgaseingangsgemisch als weiteres inertes Verdünnungsgas molekularen Stickstoff enthalten. Grundsätzlich kann das Reaktionsgaseingangsgemisch beim erfindungsgemäßen Verfahren $\geq$ 1 Vol.-%, oder $\geq$ 5 Vol.-%, oder $\geq$ 10 Vol.-%, oder $\geq$ 20 Vol.-%, oder $\geq$ 30 Vol.-%, oder $\geq$ 40 Vol.-% an molekularem Stickstoff enthalten. In der Regel wird der Gehalt des Reaktionsgaseingangsgemischs an molekularem Stickstoff jedoch bei Werten $\leq$ 80 mol-%, oder $\leq$ 70 mol-%, oder $\leq$ 60 mol-% liegen.

Auch kann das Reaktionsgaseingangsgemisch (wie bereits gesagt) Propan als inertes Verdünnungsgas enthalten. Dieser Propangehalt des Reaktionsgaseingangsgemischs kann bis zu 70 Vol.-% (z.B. 5 bis 70 Vol.-%), bzw. bis zu 60 Vol.-%, oder bis 50 Vol.-%, oder bis zu 40 Vol.-%, oder bis 30 Vol.-%, oder bis 20 Vol.-%, oder bis zu 10 Vol.-% betragen. Häufig liegt dieser Propangehalt bei $\geq$ 0,5 bzw. $\geq$ 1 Vol.-%. Er kann aber auch bei Werten von $\geq$ 0,01 Vol.-%, oder $\geq$ 0,02 Vol.-%, oder $\geq$ 0,03 Vol.-% liegen. In der Regel enthält das Reaktionsgaseingangsgemisch $\leq$ 10 Vol.-%,

vielfach ≤ 5 Vol.-% an Propan.

Dieses Propan kann beim erfindungsgemäßen Verfahren z.B. bewusst als dem Reaktionsgaseingangsgemisch separat zuzuführendes inertes Verdünnungsgas zugesetzt werden.

Selbstverständlich kann das Propan aber auch dadurch Bestandteil das Reaktionsgaseingangsgemisch werden, dass als Propenquelle für selbiges eine partielle Dehydrierung oder O-xidehydrierung von Propan fungiert (in der Regel werden diese heterogen katalysiert erfolgen). D. h., das im Reaktionsgaseingangsgemisch enthaltene Propen kann dem Reaktionsgaseingangsgemisch wenigstens teilweise unter Begleitung von nicht umgesetztem Propan aus einer partiellen Dehydrierung (z.B. homogen und/oder heterogen katalysiert, im Beisein und/oder unter Ausschluss von molekularem Sauerstoff) zugeführt werden.

Das erfindungsgemäße Verfahren umfasst insbesondere auch solche Ausführungsformen, bei denen das Reaktionsgaseingangsgemisch > 0 bis 35 Vol.-%, häufig 1 bis 25 Vol.-%, oder 5 bis 15 Vol.-%, bzw. bis 10 Vol.-% $H_2O$ enthält.

Typische Reaktionsgaseingangsgemische sind z.B. solche, die enthalten:

|  |  |  |
|---|---|---|
|  | 5 oder 6 bis 11 Vol.-% | Propen, |
|  | 2 oder 6 bis 12 Vol.-% | Wasser, |
| > 0, häufig ≥ 0,5 oder ≥ | 1 bis 10 Vol.-% | Propan, |
|  | ≥ 0 bis 5 Vol.-% | von Propen, Propan, Wasser, Sauerstoff |

und Stickstoff verschiedene Bestandteile, soviel molekularen Sauerstoff, dass das molare Verhältnis von enthaltenem molekularem Sauerstoff zu enthaltenem Propen 1 bis 3 beträgt, und als Restmenge bis zu 100 Vol.-% Gesamtmenge molekularen Stickstoff.

Erfindungsgemäße Reaktionsgaseingangsgemische können auch enthalten:

| | |
|---|---|
| 6 bis 9 Vol.-% | Propen, |
| 8 bis 18 Vol.-% | molekularer Sauerstoff, |
| 6 bis 30 bzw. bis 35 Vol.-% | Propan und |
| 32 bis 72 Vol.-% | molekularen Stickstoff. |

Erfindungsgemäße Reaktionsgaseingangsgemische können aber auch bis zu 20 Vol.-% $H_2$ enthalten.

D.h., Reaktionsgaseingangsgemische des erfindungsgemäßen Verfahrens können auch enthalten:

| | |
|---|---|
| 4 bis 25 Vol.-% | Propen, |
| 6 bis 70 Vol.-% | Propan, |
| 5 bis 60 Vol.-% | $H_2O$, |
| 8 bis 65 Vol.-% | $O_2$ und |
| 0,3 bis 20 Vol.-% | $H_2$. |

Das erfindungsgemäße Verfahren ist aber auch dann günstig, wenn das Reaktionsgaseingangsgemisch 0,1 bis 30 Vol.-% $CO_2$ enthält.

Für alle vorgenannten Konstellationen des erfindungsgemäßen Verfahrens kann sich jeweils eine zweite Reaktionsstufe der heterogen katalysierten partiellen Gasphasenoxidation von Acrolein zu Acrylsäure an das erfindungsgemäße Verfahren anschließen.

An dieser Stelle sei nochmals erwähnt, dass ein Teil des Reaktionsgaseingangsgemischs sogenanntes Kreisgas sein kann. Hierbei handelt es sich um Gas, das z.B. bei einer zweistufigen Partialoxidation von Propen zu Acrylsäure nach der Produktabtrennung (Acrylsäureabtrennung) vom Produktgasgemisch der zweiten Stufe verbleibt und bei einer Hintereinanderschaltung der beiden Stufen in der Regel zum Teil als inertes Verdünnungsgas zum Beschicken der ersten und/oder zweiten Stufe rückgeführt wird.

Typische Kreisgasgehalte sind:

0 - 0,1 Vol.-%   sonstige, z.B. Diphenyl, Diphenylether und/oder Dimethylphthalat,

(fortgesetzt)

| | |
|---|---|
| 0 - 0,1 Vol.-% | Acrylsäure, |
| 0 - 0,1 Vol.-% | Acrolein, |
| 2 - 5 Vol.-% | Sauerstoff, |
| 0,5 - 5 Vol.-% | Wasserdampf, |
| 0 - 3 Vol.-% | Kohlenmonoxid, |
| 0 - 8 Vol.-% | Kohlendioxid, |
| 0 - 2 Vol.-% | Propan, |
| 0,05 - 0,5 Vol.-% | Propen, |
| 85 - 95 Vol.-% | Stickstoff. |

Dabei kann die Acrylsäureabtrennung z.B. wie in der EP-A 982 287, der EP-A 982 289, der DE-A 199 24 532, der DE-A 101 15 277, der DE-A 196 06 877, der DE-A 197 40 252, der DE-A 196 27 847, der DE-A 100 53 086, der EP-A 982 288 und der DE-A 196 27 847 beschrieben erfolgen.

Im Übrigen sind für das erfindungsgemäße Verfahren besonders günstige Ausgestaltungen eines Zweizonenbündelreaktors auf Seite 56ff der WO 2007/082827 beschrieben (einschließlich zugehöriger Katalysatorfestbettbeschickung der Reaktionsrohre).

Damit umfasst die vorliegende Patentanmeldung insbesondere die nachfolgenden erfindungsgemäßen Ausführungsformen:

1. Verfahren zum Langzeitbetrieb einer heterogen katalysierten partiellen Gasphasenoxidation von Propen zu Acrolein, bei dem man ein Propen, molekularen Sauerstoff und wenigstens ein Inertgas enthaltendes Reaktionsgaseingangsgemisch, das den molekularen Sauerstoff und das Propen in einem molaren Verhältnis $O_2 : C_3H_6 \geq 1$ enthält, mit der Maßgabe durch ein Katalysatorfestbett, dessen Aktivmasse wenigstens ein die Elemente Mo, Fe und Bi enthaltendes Multimetalloxid ist, führt, dass

- das Katalysatorfestbett in zwei räumlich aufeinanderfolgenden Temperaturzonen A, B angeordnet ist,

- sowohl die Temperatur $T^A$ der Temperaturzone A, als auch die Temperatur $T^B$ der Temperaturzone B eine Temperatur im Temperaturbereich von 280 bis 420°C ist,

- das Reaktionsgaseingangsgemisch die Temperaturzonen A, B in der zeitlichen Abfolge "erst A" und "dann B" durchströmt, wobei sich die Temperaturzone A bis zu einem Umsatz $U^A$ des im Reaktionsgaseingangsgemisch enthaltenen Propens im Bereich von 45 bis 85 mol-% erstreckt und sich in der Temperaturzone B der Umsatz des Propens auf einen Wert $U^B \geq 90$ mol-% erhöht,

- beim einmaligen Durchgang des Reaktionsgaseingangsgemischs durch das gesamte Katalysatorfestbett die Selektivität der Acroleinbildung, bezogen auf umgesetztes Propen, $\geq 80$ mol-% beträgt,

- die Belastung des Katalysatorfestbetts mit dem im Reaktionsgaseingangsgemisch enthaltenen Propen $\geq 140$ Nl Propen/l Katalysatorfestbett·h beträgt,

- die Temperaturen $T^A$ und $T^B$ nach erfolgter frischer Beschickung des Katalysatorfestbetts so beschaffen sind, dass die Differenz $\Delta T^{BA} = T^B - T^A > 0°C$ beträgt,

- man anschließend mit zunehmender Betriebsdauer, um der Minderung der Qualität des Katalysatorfestbetts entgegenzuwirken, wenigstens eine der beiden Temperaturen $T^A$, $T^B$ erhöht, dadurch gekennzeichnet, dass

- die Erhöhung der wenigstens einen der beiden Temperaturen $T^A$, $T^B$ so vorgenommen wird, dass sich die Differenz $\Delta T^{BA} = T^B - T^A$ mit zunehmender Betriebsdauer erhöht.

2. Verfahren gemäß Ausführungsform 1, dadurch gekennzeichnet, dass nach erfolgter frischer Beschickung des Katalysatorfestbetts $\Delta T^{BA} \geq 2°C$ beträgt.

3. Verfahren gemäß Ausführungsform 1, dadurch gekennzeichnet, dass nach erfolgter frischer Beschickung des Katalysatorfestbetts $\Delta T^{BA} \geq 3°C$ beträgt.

4. Verfahren gemäß Ausführungsform 1, dadurch gekennzeichnet, dass nach erfolgter frischer Beschickung des Kata-

lysatorfestbetts $\Delta T^{BA} \geq 5°C$ beträgt.

5. Verfahren gemäß einer der Ausführungsformen 1 bis 4, dadurch gekennzeichnet, dass nach erfolgter frischer Beschickung des Katalysatorfestbetts $\Delta T^{BA} \leq 50°C$ beträgt.

6. Verfahren gemäß einer der Ausführungsformen 1 bis 4, dadurch gekennzeichnet, dass nach erfolgter frischer Beschickung des Katalysatorfestbetts $\Delta T^{BA} \leq 40°C$ beträgt.

7. Verfahren gemäß Ausführungsform 1, dadurch gekennzeichnet, dass nach erfolgter frischer Beschickung des Katalysatorfestbetts $\Delta T^{BA} \geq 5°C$ und $\leq 35°C$ beträgt.

8. Verfahren gemäß Ausführungsform 1, dadurch gekennzeichnet, dass nach erfolgter frischer Beschickung des Katalysatorfestbetts $\Delta T^{BA} \geq 5°C$ und $\leq 30°C$ beträgt.

9. Verfahren gemäß Ausführungsform 1, dadurch gekennzeichnet, dass nach erfolgter frischer Beschickung des Katalysatorfestbetts $\Delta T^{BA} \geq 10°C$ und $\leq 25°C$ beträgt.

10. Verfahren gemäß einer der Ausführungsformen 1 bis 9, dadurch gekennzeichnet, dass nach erfolgter frischer Beschickung des Katalysatorfestbetts der Unterschied zwischen der maximalen Reaktionstemperatur in der Temperaturzone A, $T^{maxA}$, und der maximalen Reaktionstemperatur in der Temperaturzone B, $T^{maxB}$, gebildet als $T^{maxA} - T^{maxB}$, $\geq 0°C$ und $\leq 80°C$ beträgt.

11. Verfahren gemäß Ausführungsform 10, dadurch gekennzeichnet, dass $T^{maxA} - T^{maxB} \geq 0°C$ und $\leq 70°C$ beträgt.

12. Verfahren gemäß Ausführungsform 10, dadurch gekennzeichnet, dass $T^{maxA} - T^{maxB} \geq 2°C$ und $\leq 40°C$ beträgt.

13. Verfahren gemäß Ausführungsform 10, dadurch gekennzeichnet, dass $T^{maxA} - T^{maxB} \geq 5°C$ und $\leq 25°C$ beträgt.

14. Verfahren gemäß Ausführungsform 10, dadurch gekennzeichnet, dass $T^{maxA} - T^{maxB} \geq 10°C$ und $\leq 25°C$ beträgt.

15. Verfahren gemäß einer der Ausführungsformen 1 bis 14, dadurch gekennzeichnet, dass im Langzeitbetrieb sowohl $T^B$ als auch $T^A$ erhöht wird.

16. Verfahren gemäß Ausführungsform 15, dadurch gekennzeichnet, dass, bezogen auf die selbe Betriebsdauer im Langzeitbetrieb, die Erhöhung von $T^B$ das 1,2- bis 5-fache der Erhöhung von $T^A$ beträgt.

17. Verfahren gemäß Ausführungsform 15, dadurch gekennzeichnet, dass, bezogen auf die selbe Betriebsdauer im Langzeitbetrieb, die Erhöhung von $T^B$ das 1,5- bis 3-fache der Erhöhung von $T^A$ beträgt.

18. Verfahren gemäß Ausführungsform 15, dadurch gekennzeichnet, dass, bezogen auf die selbe Betriebsdauer im Langzeitbetrieb, die Erhöhung von $T^B$ das 1,5- bis 2,5-fache der Erhöhung von $T^A$ beträgt.

19. Verfahren gemäß einer der Ausführungsformen 1 bis 18, dadurch gekennzeichnet, dass sich der Unterschied zwischen der maximalen Reaktionstemperatur in der Temperaturzone A, $T^{maxA}$, und der maximalen Reaktionstemperatur in der Temperaturzone B, $T^{maxB}$, gebildet als $T^{maxA} - T^{maxB}$, mit zunehmender Betriebsdauer verringert, aber nicht negativ wird.

20. Verfahren gemäß einer der Ausführungsformen 1 bis 19, dadurch gekennzeichnet, dass sich die Betriebsdauer auf wenigstens 6 Monate erstreckt.

21. Verfahren gemäß einer der Ausführungsformen 1 bis 19, dadurch gekennzeichnet, dass sich die Betriebsdauer auf wenigstens 12 Monate erstreckt.

22. Verfahren gemäß einer der Ausführungsformen 1 bis 19, dadurch gekennzeichnet, dass sich die Betriebsdauer auf wenigstens 18 Monate erstreckt.

23. Verfahren gemäß einer der Ausführungsformen 1 bis 19, dadurch gekennzeichnet, dass sich die Betriebsdauer auf wenigstens 24 Monate erstreckt.

24. Verfahren gemäß einer der Ausführungsformen 1 bis 19, dadurch gekennzeichnet, dass sich die Betriebsdauer auf wenigstens 36 Monate erstreckt.

25. Verfahren gemäß einer der Ausführungsformen 1 bis 24, dadurch gekennzeichnet, dass $\Delta T^{BA}$ mit zunehmender Betriebsdauer 70°C nicht überschreitet.

26. Verfahren gemäß Ausführungsform 25, dadurch gekennzeichnet, dass $\Delta T^{BA}$ mit zunehmender Betriebsdauer 60°C nicht überschreitet.

27. Verfahren gemäß Ausführungsform 25, dadurch gekennzeichnet, dass $\Delta T^{BA}$ mit zunehmender Betriebsdauer 50°C nicht überschreitet.

28. Verfahren gemäß Ausführungsform 25, dadurch gekennzeichnet, dass $\Delta T^{BA}$ mit zunehmender Betriebsdauer 40°C nicht überschreitet.

29. Verfahren gemäß einer der Ausführungsformen 1 bis 28, dadurch gekennzeichnet, dass $T^A$ über die gesamte Betriebsdauer im Bereich von 300 bis 400°C liegt.

30. Verfahren gemäß einer der Ausführungsformen 1 bis 28, dadurch gekennzeichnet, dass $T^A$ über die gesamte Betriebsdauer im Bereich von 310 bis 390°C liegt.

31. Verfahren gemäß einer der Ausführungsformen 1 bis 28, dadurch gekennzeichnet, dass $T^A$ über die gesamte Betriebsdauer im Bereich von 320 bis 380°C liegt.

32. Verfahren gemäß einer der Ausführungsformen 1 bis 31, dadurch gekennzeichnet, dass $T^B$ über die gesamte Betriebsdauer im Bereich von 305 bis 415°C liegt.

33. Verfahren gemäß einer der Ausführungsformen 1 bis 31, dadurch gekennzeichnet, dass $T^B$ über die gesamte Betriebsdauer im Bereich von 315 bis 410°C liegt.

34. Verfahren gemäß einer der Ausführungsformen 1 bis 31, dadurch gekennzeichnet, dass $T^B$ über die gesamte Betriebsdauer im Bereich von 330 bis 410°C liegt.

35. Verfahren gemäß einer der Ausführungsformen 1 bis 34, dadurch gekennzeichnet, dass sich die Temperaturzone A bis zu einem Umsatz $U^A$ des im Reaktionsgaseingangsgemisch enthaltenen Propens im Bereich von 50 bis 80 mol-% erstreckt.

36. Verfahren gemäß einer der Ausführungsformen 1 bis 34, dadurch gekennzeichnet, dass sich die Temperaturzone A bis zu einem Umsatz $U^A$ des im Reaktionsgaseingangsgemisch enthaltenen Propens im Bereich von 55 bis 75 mol-% erstreckt.

37. Verfahren gemäß einer der Ausführungsformen 1 bis 36, dadurch gekennzeichnet, dass sich in der Temperaturzone B der Umsatz des Propens auf einen Wert $\geq$ 92 mol-% erhöht.

38. Verfahren gemäß einer der Ausführungsformen 1 bis 36, dadurch gekennzeichnet, dass sich in der Temperaturzone B der Umsatz des Propens auf einen Wert $\geq$ 94 mol-% erhöht.

39. Verfahren gemäß einer der Ausführungsformen 1 bis 36, dadurch gekennzeichnet, dass sich in der Temperaturzone B der Umsatz des Propens auf einen Wert $\geq$ 96 mol-% erhöht.

40. Verfahren gemäß einer der Ausführungsformen 1 bis 39, dadurch gekennzeichnet, dass die Selektivität der Acroleinbildung beim einmaligen Durchgang des Reaktionsgaseingangsgemischs durch das gesamte Katalysatorfestbett und bezogen auf umgesetztes Propen $\geq$ 85 mol-% oder $\geq$ 89 mol-% beträgt.

41. Verfahren gemäß einer der Ausführungsformen 1 bis 40, dadurch gekennzeichnet, dass die Belastung des Katalysatorfestbetts mit Propen $\geq$ 150 Nl/l·h beträgt.

42. Verfahren gemäß einer der Ausführungsformen 1 bis 40, dadurch gekennzeichnet, dass die Belastung des Katalysatorfestbetts mit Propen $\geq$ 160 Nl/l·h beträgt.

43. Verfahren gemäß einer der Ausführungsformen 1 bis 40, dadurch gekennzeichnet, dass die Belastung des Katalysatorfestbetts mit Propen $\geq$ 170 Nl/l·h beträgt.

44. Verfahren gemäß einer der Ausführungsformen 1 bis 40, dadurch gekennzeichnet, dass die Belastung des Katalysatorfestbetts mit Propen $\geq$ 180 Nl/l·h beträgt.

45. Verfahren gemäß einer der Ausführungsformen 1 bis 44, dadurch gekennzeichnet, dass die Belastung des Katalysatorfestbetts mit Propen $\leq$ 400 Nl/l·h beträgt.

46. Verfahren gemäß einer der Ausführungsformen 1 bis 44, dadurch gekennzeichnet, dass die Belastung des Katalysatorfestbetts mit Propen $\leq$ 300 Nl/l·h beträgt.

47. Verfahren gemäß einer der Ausführungsformen 1 bis 44, dadurch gekennzeichnet, dass die Belastung des Katalysatorfestbetts mit Propen $\leq$ 250 Nl/l·h beträgt.

48. Verfahren gemäß einer der Ausführungsformen 1 bis 47, dadurch gekennzeichnet, dass sich das Katalysatorfestbett in den Reaktionsrohren eines zwei Temperaturzonen aufweisenden Rohrbündelreaktors befindet.

49. Verfahren gemäß einer der Ausführungsformen 1 bis 48, dadurch gekennzeichnet, dass man den Langzeitbetrieb unterbricht und das Katalysatorfestbett durch Durchleiten eines heißen Gasgemischs aus molekularem Sauerstoff und Inertgas durch das Katalysatorfestbett regeneriert und daran anschließend den Langzeitbetrieb fortsetzt.

50. Verfahren gemäß einer der Ausführungsformen 1 bis 49, dadurch gekennzeichnet, dass das Reaktionsgaseingangsgemisch 2 bis 25 Vol.-% Propen enthält.

51. Verfahren gemäß einer der Ausführungsformen 1 bis 49, dadurch gekennzeichnet, dass das Reaktionsgaseingangsgemisch 4 bis 15 Vol.-% Propen enthält.

52. Verfahren gemäß einer der Ausführungsformen 1 bis 49, dadurch gekennzeichnet, dass das Reaktionsgaseingangsgemisch 5 bis 12 Vol.-% Propen enthält.

53. Verfahren gemäß einer der Ausführungsformen 1 bis 52, dadurch gekennzeichnet, dass das wenigstens eine Inertgas zu wenigstens 40% seines Volumens aus molekularem Stickstoff besteht.

54. Verfahren gemäß einer der Ausführungsformen 1 bis 52, dadurch gekennzeichnet, dass das wenigstens eine Inertgas zu bis zu 50% seines Volumens aus n-Propan besteht.

55. Verfahren gemäß einer der Ausführungsformen 1 bis 49, dadurch gekennzeichnet, dass das Reaktionsgaseingangsgemisch zu

| | |
|---|---|
| 5 bis 15 Vol.-% | aus Propen, |
| 0,5 bis 20 Vol.-% | aus Wasser, |
| $\geq$ 0 bis 10 Vol.-% | aus von Propen, Wasser, molekularem Sauerstoff und molekularem Stickstoff verschiedenen Bestandteilen, |

soviel molekularem Sauerstoff, dass das molare Verhältnis von im Reaktionsgaseingangsgemisch enthaltenem molekularem Sauerstoff zu darin enthaltenem Propen 1,5 bis 2,5 beträgt, und

als Restmenge bis zu 100 Vol.-% Gesamtmenge aus molekularem Stickstoff zusammengesetzt ist.

56. Verfahren gemäß einer der Ausführungsformen 1 bis 54, dadurch gekennzeichnet, dass das Reaktionsgaseingangsgemisch den molekularen Sauerstoff und das Propen in einem molaren Verhältnis $O_2 : C_3H_6 \leq 3$ enthält.

57. Verfahren gemäß einer der Ausführungsformen 1 bis 54, dadurch gekennzeichnet, dass das Reaktionsgaseingangsgemisch den molekularen Sauerstoff und das Propen in einem molaren Verhältnis $O_2 : C_3H_6 \geq 1,2$ und $\leq 3$ enthält.

58. Verfahren gemäß einer der Ausführungsformen 1 bis 57, dadurch gekennzeichnet, dass die Aktivmasse des Katalysatorfestbetts wenigstens ein die Elemente Mo, Fe und Bi, sowie zusätzlich wenigstens eines der beiden Elemente Ni und Co enthaltendes Multimetalloxid ist.

59. Verfahren gemäß Ausführungsform 58, dadurch gekennzeichnet, dass von den fünf Elementen Mo, Fe, Bi, Ni und Co, bezogen auf ihre in der Aktivmasse enthaltene molare Gesamtmenge, auf das Element Mo der größte molare Anteil in mol-% und bezogen auf die molare Gesamtmenge G entfällt.

60. Verfahren gemäß einer der Ausführungsformen 1 bis 57, dadurch gekennzeichnet, dass die Aktivmasse wenigstens eine Multimetalloxidaktivmasse der allgemeinen Formel I

$$Mo_{12}Bi_aFe_bX^1_cX^2_dX^3_eX^4_fO_n \qquad (I),$$

in der die Variablen nachfolgende Bedeutung aufweisen:

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,
a = 0,5 bis 5,
b = 0,01 bis 5,
c = 0 bis 10,
d = 0 bis 2,
e = 0 bis 8,
f = 0 bis 10, und
n = eine Zahl, die durch die Wertigkeit und Häufigkeit der von Sauerstoff verschiedenen Elemente in I bestimmt wird,

ist.

61. Verfahren gemäß Ausführungsform 60, dadurch gekennzeichnet, dass der stöchiometrische Koeffizient b 2 bis 4 ist.

62. Verfahren gemäß Ausführungsform 60 oder 61, dadurch gekennzeichnet, dass der stöchiometrische Koeffizient c 3 bis 10 ist.

63. Verfahren gemäß einer der Ausführungsformen 60 bis 61, dadurch gekennzeichnet, dass der stöchiometrische Koeffizient d 0,02 bis 2 ist.

64. Verfahren gemäß einer der Ausführungsformen 1 bis 63, dadurch gekennzeichnet, dass sich $\Delta T^{BA}$ mit zunehmender Betriebsdauer um wenigstens 5°C erhöht.

65. Verfahren gemäß einer der Ausführungsformen 1 bis 63, dadurch gekennzeichnet, dass sich $\Delta T^{BA}$ mit zunehmender Betriebsdauer um wenigstens 10°C erhöht.

66. Verfahren gemäß einer der Ausführungsformen 1 bis 65, dadurch gekennzeichnet, dass sich $\Delta T^{BA}$ mit zunehmender Betriebsdauer um nicht mehr als 30°C erhöht.

67. Verfahren gemäß einer der Ausführungsformen 1 bis 65, dadurch gekennzeichnet, dass sich $\Delta T^{BA}$ mit zunehmender Betriebsdauer um nicht mehr als 20°C erhöht.

68. Verfahren gemäß einer der Ausführungsformen 1 bis 67, dadurch gekennzeichnet, dass die maximale Reaktionstemperatur in der Reaktionszone A, $T^{maxA}$, mit zunehmender Betriebsdauer 420°C nicht überschreitet.

69. Verfahren gemäß einer der Ausführungsformen 1 bis 67, dadurch gekennzeichnet, dass die maximale Reaktionstemperatur in der Reaktionszone A, $T^{maxA}$, mit zunehmender Betriebsdauer 410°C nicht überschreitet.

70. Verfahren gemäß einer der Ausführungsformen 1 bis 69, dadurch gekennzeichnet, dass die maximale Reaktionstemperatur in der Reaktionszone A, $T^{maxA}$, $\geq 350$°C beträgt.

71. Verfahren gemäß einer der Ausführungsformen 1 bis 69, dadurch gekennzeichnet, dass die maximale Reaktionstemperatur in der Reaktionszone A, $T^{maxA}$, $\geq 370$°C beträgt.

72. Verfahren gemäß einer der Ausführungsformen 1 bis 71, dadurch gekennzeichnet, dass die Differenz zwischen der maximalen Reaktionstemperatur in der Temperaturzone A, $T^{maxA}$, und der Temperatur $T^A$ der Temperaturzone A, gebildet als $\Delta T^{HB}A = T^{maxA} - T^A$, 40 bis 90°C beträgt.

73. Verfahren gemäß einer der Ausführungsformen 1 bis 72, dadurch gekennzeichnet, dass sich das Katalysatorfestbett in den Rohren eines zwei Temperaturzonen aufweisenden Rohrbündelreaktors befindet, wobei jede Temperaturzone von einer Salzschmelze durchströmt wird und der Wärmeübergang von der Rohrwand zur Salzschmelze $\geq 1000$ W/m²·K

und ≤ 3000 W/m$^2$·K beträgt.

74. Verfahren gemäß einer der Ausführungsformen 1 bis 72, dadurch gekennzeichnet, dass sich das Katalysatorfestbett in den Rohren eines zwei Temperaturzonen aufweisenden Rohrbündelreaktors befindet, wobei jede Temperaturzone von einer Salzschmelze durchströmt wird und der Wärmeübergang von der Rohrwand zur Salzschmelze ≥ 1500 W/m$^2$·K und ≤ 3000 W/m$^2$·K beträgt.

75. Verfahren gemäß einer der Ausführungsformen 1 bis 72, dadurch gekennzeichnet, dass sich das Katalysatorfestbett in den Rohren eines zwei Temperaturzonen aufweisenden Rohrbündelreaktors befindet, wobei jede Temperaturzone von einer Salzschmelze durchströmt wird und der Wärmeübergang von der Rohrwand zur Salzschmelze ≥ 2000 W/m$^2$·K und ≤ 3000 W/m$^2$·K beträgt.

Beispiel und Vergleichsbeispiel

I. Beispiel

Ein Thermoreaktionsrohr (V2A Stahl; 33,7 mm Außendurchmesser, 2 mm Wandstärke, 29,7 mm Innendurchmesser, Länge: 350 cm, sowie ein in der Thermorohrmitte zentriert von oben nach unten verlaufendes Thermoschutzrohr (Thermohülse) zur Aufnahme eines Thermoelements, mit dem auf der gesamten Rohrlänge die Reaktionstemperatur (die effektive Katalysatorfestbetttemperatur ermittelt werden konnte) wurde von oben nach unten wie folgt beschickt:

Abschnitt 1:    50 cm Länge

Steatitringe der Geometrie 7 mm x 7 mm x 4 mm (Außendurchmesser x Länge x Innendurchmesser, Steatit C220 der Fa. CeramTec) als Vorschüttung.

Abschnitt 2:    140 cm Länge

Katalysatorbeschickung mit einem homogenen Gemisch bestehend aus 30 Gew.-% an Steatitringen der Geometrie 5 mm x 3 mm x 2 mm (Außendurchmesser x Länge x Innendurchmesser; Steatit C220 der Fa. CeramTec), 55 Gew.-% Vollkatalysator aus Abschnitt 3 und 15 Gew.-% versplittetem Vollkatalysator aus Abschnitt 3.

Abschnitt 3:    160 cm Länge

Katalysatorbeschickung mit einem homogenen Gemisch aus 85 Gew.-% des ringförmigen Vollkatalysators I (5 mm x 3 mm x 2 mm = Außendurchmesser x Länge x Innendurchmesser) gemäß Ausführungsbeispiel der DE-A 102009047291 mit der Stöchiometrie $[Bi_2W_2O_9 \cdot 2WO_3]_{0,40}$ $[Mo_{12}Co_{5,4}Fe_{3,1}Si_{1,5}K_{0,08}O_x]_1$, und 15 Gew.-% Splitt des vorgenannten Vollkatalysators

Das Thermoreaktionsrohr soll das Verhalten eines bloßen Reaktionsrohres aus entsprechendem Material und mit identischer Rohrlänge abbilden, dessen Innendurchmesser bei 2 mm Wandstärke 26 mm beträgt und das in entsprechender Weise wie das Thermoreaktionsrohr beschickt wird, sieht man davon ab, dass der im Fall des Thermoreaktionsrohrs im jeweiligen Abschnitt jeweils versplittete Gewichtsanteil an Vollkatalysatorringen beim bloßen Reaktionsrohr unversplittet, d.h. in Ringform in die Beschickung mit eingeht.

Von oben nach unten wurden die ersten 175 cm des Thermoreaktionsrohrs mittels eines über die 175 cm zum Reaktionsgasgemisch im Gegenstrom gepumpten Salzbades A thermostatisiert, das mit der Temperatur T$^A$ zugeführt wurde. Die zweiten 175 wurden mittels eines in entsprechender Weise im Gegenstrom gepumpten Salzbades B thermostatisiert, das mit der Temperatur T$^B$ zugeführt wurde. Über die jeweilige Temperaturzone war die jeweilige Salzbadtemperatur im Wesentlichen konstant.

Die beiden Salzbäder A, B bestanden jeweils aus 53 Gew.-% Kaliumnitrat, 40 Gew.-% Natriumnitrit und 7 Gew.-% Natriumnitrat. In den beiden 175 cm langen Rohrabschnitten erfolgte die Anströmung des Reaktionsrohres im Wesentlichen vertikal zur Strömungsrichtung des Reaktionsgases. Die Strömungsgeschwindigkeit beider Salzschmelzen war so bemessen, dass eine weitere Erhöhung im Wesentlichen keine Verbesserung des Wärmedurchgangs aus dem Thermorohrinneren ins Salzbad bewirkt hätte (vgl. EP-A1547994).

Das wie vorstehend beschrieben beschickte Thermorohr wurde im stationären Betrieb mit einem Reaktionsgaseingangsgemisch beschickt, das nachfolgende Gehalte aufwies und aus Luft (als Sauerstoffquelle), chemical grade Propen und Kreisgas erzeugt wurde:

6 bis 6,5 Vol.-%        Propen

(fortgesetzt)

| | |
|---|---|
| 0,7 bis 1,2 Vol.-% | $H_2O$, |
| 0,4 bis 0,6 Vol.% | CO, |
| 0,7 bis 1,1 Vol.-% | $CO_2$, |
| 0,01 bis 0,04 Vol.-% | Acrolein, |
| 0,005 bis 0,015 Vol.-% | Ethen, |
| 0,025 bis 0,035 Vol.-% | Propan, |
| 10,8 bis 11,7 Vol.-% | $O_2$ und |
| wenigstens 77 Vol-% | $N_2$. |

Die Belastung des Katalysatorfestbetts mit im Reaktionsgaseingangsgemisch enthaltenem Propen lag während der stationären Betriebsphasen stets im Intervall 185 ± 10 Nl/l·h. Der Druck am Eingang des Thermoreaktionsrohres betrug dabei im Wesentlichen 2,0 atm.

Während einer stationären Betriebsphase wurden die Temperaturen $T^A$, $T^B$ jeweils konstant gehalten.

Eine stationäre Betriebsphase erstreckte sich über 24 Betriebstage. Am Ende einer stationären Betriebsphase wurde das Partialoxidationsverfahren jeweils unterbrochen und das Katalysatorfestbett wie in der WO 2004/085369 beschrieben regeneriert.

Zur Wiederinbetriebnahme des Partialoxidationsverfahrens nach einer erfolgten Regenerierung des Katalysatorfestbetts sowie zur Erstinbetriebnahme des Katalysatorfestbetts wurden die Gehalte des Reaktionsgaseingangsgemischs auf folgende Werte verändert:

| | |
|---|---|
| 5,7 bis 6,2 Vol.-% | Propen |
| 0,7 bis 1,2 Vol.-% | $H_2O$, |
| 0,4 bis 0,6 Vol.% | CO, |
| 0,7 bis 1,1 Vol.-% | $CO_2$, |
| 0,01 bis 0,04 Vol.-% | Acrolein, |
| 0,005 bis 0,015 Vol.-% | Ethen, |
| 0,025 bis 0,035 Vol.-% | Propan, |
| 10,3 bis 11,2 Vol.-% | $O_2$ und |
| wenigstens 78,5 Vol-% | $N_2$. |

Zusätzlich wurde für eine Wiederinbetriebnahme sowie zur Erstinbetriebnahme die Propenbelastung des Katalysatorfestbetts auf 100 Nl/l·h abgesenkt und $T^A$, $T^B$ unter der Maßgabe $\Delta T^{BA} \geq 0$ soweit beschränkt, dass der auf einen Einmaldurchgang des Reaktionsgasgemischs durch das Thermorohr bezogene Propenumsatz auf 94 mol-% begrenzt war. Von diesen Betriebsbedingungen ausgehend wurden die Betriebsbedingungen des stationären Betriebs so angesteuert, dass $T^{maxA}$ stets > als $T^{maxB}$ sowie stets kleiner als das $T^{maxA}$ im sich anschließenden stationären Betrieb war.

Die nachfolgende Tabelle 1 zeigt die in Abhängigkeit von $T^A$ und $T^B$ für die jeweilige stationäre Betriebsphase bei der jeweiligen Propenbelastung PL des Katalysatorfestbetts resultierenden Werte für $T^{maxA}$ sowie für die Gesamtselektivität $S^{AC+AA}$ der Bildung an Acrolein und Acrylsäure (die Selektivität der Gesamtzielproduktbildung bezogen auf beim Einmaldurchgang des Reaktionsgasgemischs durch das Thermorohr (Thermoreaktionsrohr) umgesetztes Propen). Ferner zeigt die Tabelle 1 die Werte für $\Delta T^{BA}$ und für $\Delta T^{HB}{}_A$. Alle Angaben beziehen sich jeweils auf den 24. Betriebstag der jeweiligen stationären Betriebsphase.

II. Vergleichsbeispiel

Alles im Vergleichsbeispiel wurde wie im Beispiel durchgeführt, jedoch mit dem Unterschied, dass im Langzeitbetrieb zum Beibehalt des Propenumsatzes $T^A$, $T^B$ so verändert wurden, dass sich $\Delta T^{BA}$ gemäß der Lehre der WO 2007/082827 verringerte. Tabelle 2 zeigt die resultierenden Ergebnisse in entsprechender Weise wie dies Tabelle 1 für das Beispiel tut.

Tabelle 1 (Beispiel):

| Betriebsphase | PL (Nl/l·h) | $T^A$ (°C) | $T^B$ (°C) | $T^{maxA}$ (°C) | $\Delta T^{BA}$ (°C) | $\Delta T^{HB}_A$ (°C) | $S^{AC+AA}$ (mol-%) |
|---|---|---|---|---|---|---|---|
| 1 | 180 | 325 | 341 | 385 | 16 | 60 | 95,5 |
| 2 | 175 | 325 | 342 | 384 | 17 | 59 | 96,2 |
| 3 | 175 | 325 | 343 | 384 | 18 | 59 | 96,5 |
| 4 | 190 | 327 | 346 | 385 | 19 | 58 | 96,1 |
| 5 | 179 | 328 | 347 | 386 | 19 | 58 | 96,1 |
| 6 | 182 | 329 | 348 | 392 | 19 | 63 | 96,2 |
| 7 | 175 | 330 | 349 | 394 | 19 | 64 | 96,3 |
| 8 | 192 | 333 | 353 | 396 | 20 | 62 | 96,5 |
| 9 | 192 | 333 | 355 | 396 | 22 | 63 | 96,3 |
| 10 | 194 | 335 | 357 | 402 | 22 | 67 | 96,5 |
| 11 | 187 | 336 | 359 | 400 | 23 | 64 | 96,5 |
| 12 | 182 | 334 | 357 | 396 | 23 | 62 | 96,5 |
| 13 | 188 | 336 | 360 | 398 | 24 | 62 | 96,5 |
| 14 | 187 | 334 | 358 | 400 | 24 | 66 | 96,3 |
| 15 | 188 | 336 | 361 | 405 | 25 | 69 | 96,6 |
| 16 | 183 | 338 | 363 | 404 | 25 | 66 | 96,3 |
| 17 | 190 | 340 | 365 | 409 | 25 | 69 | 96,1 |
| 18 | 184 | 340 | 368 | 408 | 28 | 68 | 96,4 |
| 19 | 190 | 337 | 365 | 406 | 28 | 69 | 96,4 |
| 20 | 192 | 340 | 369 | 409 | 29 | 69 | 96,4 |
| 22 | 190 | 341 | 370 | 408 | 29 | 67 | 96,3 |
| 23 | 194 | 341 | 370 | 410 | 29 | 69 | 96,4 |
| 24 | 186 | 343 | 373 | 410 | 30 | 67 | 95,8 |
| 25 | 191 | 342 | 372 | 411 | 30 | 69 | 95,9 |
| 26 | 195 | 341 | 372 | 408 | 31 | 67 | 95,5 |
| 29 | 185 | 340 | 372 | 402 | 32 | 62 | 95,4 |
| 34 | 191 | 349 | 382 | 411 | 33 | 62 | 94,7 |
| 37 | 189 | 355 | 389 | 415 | 34 | 60 | 92,8 |
| 38 | 194 | 360 | 393 | 418 | 33 | 58 | 92,4 |

Nach der 38. Betriebsphase wurde das Katalysatorfestbett gegen ein frisches Katalysatorfestbett ausgetauscht.

Bis zum Ende der 23. Betriebsphase betrug der auf einen Einmaldurchgang des Reaktionsgasgemischs durch das Thermorohr bezogene resultierende Propenumsatz 95,1 ± 0,3 mol%. Der Propenumsatz in der Temperaturzone A lag während der gesamten Betriebsphasen im Bereich von 60 bis 72 mol-%.

Am Ende der 38. Betriebsphase betrug der auf einen Einmaldurchgang des Reaktionsgasgemischs durch das Thermorohr bezogene resultierende Propenumsatz nur noch 91,8 mol-%.

$T^{maxB}$ war in allen Betriebsphasen kleiner als $T^{maxA}$. Bei Beginn des Langzeitbetriebs betrug die Differenz $T^{maxB} - T^{maxA}$ 15°C und verringerte sich im Verlauf des Langzeitbetriebs auf 1,5°C. Die Selektivität der Acroleinbildung, bezogen auf umgesetztes Propen, war in allen Betriebsphasen > 85 mol%.

Tabelle 2 (Vergleichsbeispiel):

| Betriebsphase | PL (Nl/l·h) | $T^A$ (°C) | $T^B$ (°C) | $T^{maxA}$ (°C) | $\Delta T^{BA}$ (°C) | $\Delta T^{HB}_A$ (°C) | $S^{AC+AA}$ (mol-%) |
|---|---|---|---|---|---|---|---|
| 1 | 188 | 325 | 345 | 387 | 20 | 62 | 95,5 |
| 2 | 185 | 326 | 346 | 394 | 20 | 68 | 96,0 |
| 3 | 194 | 327 | 346 | 396 | 19 | 69 | 96,3 |

Nach der 15. Betriebsphase wurde das Katalysatorfestbett gegen ein frisches Katalysatorfestbett ausgetauscht. Bis zum Ende der 12. Betriebsphase betrug der auf einen Einmaldurchgang des Reaktionsgasgemischs durch das Thermorohr bezogene resultierende Propenumsatz 94,8 ± 0,3 mol-%. Der Propenumsatz in der Temperaturzone A lag während der gesamten Betriebsphase im Bereich 65 bis 77 mol-%.

Am Ende der 15. Betriebsphase betrug der auf einen Einmaldurchgang des Reaktionsgasgemischs durch das Thermorohr bezogene resultierende Propenumsatz nur noch 92 mol-%.

$T^{maxB}$ war in allen Betriebsphasen kleiner als $T^{maxA}$.

Die Selektivität der Acroleinbildung, bezogen auf umgesetztes Propen, war in allen Betriebsphasen > 85 mol-%.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Verfahren zum Langzeitbetrieb einer heterogen katalysierten partiellen Gasphasenoxidation von Propen zu Acrolein, bei dem man ein Propen, molekularen Sauerstoff und wenigstens ein Inertgas enthaltendes Reaktionsgaseingangsgemisch, das den molekularen Sauerstoff und das Propen in einem molaren Verhältnis $O_2 : C_3H_6 \geq 1$ enthält, mit der Maßgabe durch ein Katalysatorfestbett, dessen Aktivmasse wenigstens ein die Elemente Mo, Fe und Bi enthaltendes Multimetalloxid ist, führt, dass

- das Katalysatorfestbett in zwei räumlich aufeinanderfolgenden Temperaturzonen A, B angeordnet ist,
- sowohl die Temperatur $T^A$ der Temperaturzone A, als auch die Temperatur $T^B$ der Temperaturzone B eine Temperatur im Temperaturbereich von 280 bis 420°C ist,
- das Reaktionsgaseingangsgemisch die Temperaturzonen A, B in der zeitlichen Abfolge "erst A" und "dann B" durchströmt, wobei sich die Temperaturzone A bis zu einem Umsatz $U^A$ des im Reaktionsgaseingangsgemisch enthaltenen Propens im Bereich von 45 bis 85 mol-% erstreckt und sich in der Temperaturzone B der Umsatz des Propens auf einen Wert $U^B \geq 90$ mol-% erhöht,
- beim einmaligen Durchgang des Reaktionsgaseingangsgemischs durch das gesamte Katalysatorfestbett die Selektivität der Acroleinbildung, bezogen auf umgesetztes Propen, $\geq 80$ mol-% beträgt,
- die Belastung des Katalysatorfestbetts mit dem im Reaktionsgaseingangsgemisch enthaltenen Propen $\geq 140$ Nl Propen/l Katalysatorfestbett·h beträgt,
- die Temperaturen $T^A$ und $T^B$ nach erfolgter frischer Beschickung des Katalysatorfestbetts so beschaffen sind, dass die Differenz $\Delta T^{BA} = T^B - T^A > 0°C$ beträgt,
- man anschließend mit zunehmender Betriebsdauer, um der Minderung der Qualität des Katalysatorfestbetts entgegenzuwirken, wenigstens eine der beiden Temperaturen $T^A$, $T^B$ erhöht, **dadurch gekennzeichnet, dass**
- die Erhöhung der wenigstens einen der beiden Temperaturen $T^A$, $T^B$ so vorgenommen wird, dass sich die Differenz $\Delta T^{BA} = T^B - T^A$ mit zunehmender Betriebsdauer erhöht.

2. Verfahren nach Anspruch 1, **dadurch gekennzeichnet, dass** nach erfolgter frischer Beschickung des Katalysatorfestbetts $\Delta T^{BA} \geq 2°C$ beträgt.

3. Verfahren nach Anspruch 1 oder 2, **dadurch gekennzeichnet, dass** nach erfolgter frischer Beschickung des Katalysatorfestbetts $\Delta T^{BA} \leq 50°C$ beträgt.

4. Verfahren nach einem der Ansprüche 1 bis 3, **dadurch gekennzeichnet, dass** nach erfolgter frischer Beschickung des Katalysatorfestbetts der Unterschied zwischen der maximalen Reaktionstemperatur in der Temperaturzone A, $T^{maxA}$, und der maximalen Reaktionstemperatur in der Temperaturzone B, $T^{maxB}$, gebildet als $T^{maxA} - T^{maxB}$, $\geq 0°C$

und $\leq 80°C$ beträgt.

5. Verfahren nach einem der Ansprüche 1 bis 4, **dadurch gekennzeichnet, dass** im Langzeitbetrieb sowohl $T^B$ als auch $T^A$ erhöht wird.

6. Verfahren nach Anspruch 5, **dadurch gekennzeichnet, dass**, bezogen auf die selbe Betriebsdauer im Langzeitbetrieb, die Erhöhung von $T^B$ das 1,2- bis 5-fache der Erhöhung von $T^A$ beträgt.

7. Verfahren nach einem der Ansprüche 1 bis 6, **dadurch gekennzeichnet, dass** sich der Unterschied zwischen der maximalen Reaktionstemperatur in der Temperaturzone A, $T^{maxA}$, und der maximalen Reaktionstemperatur in der Temperaturzone B, $T^{maxB}$, gebildet als $T^{maxA} - T^{maxB}$, mit zunehmender Betriebsdauer verringert, aber nicht negativ wird.

8. Verfahren nach einem der Ansprüche 1 bis 7, **dadurch gekennzeichnet, dass** sich die Betriebsdauer auf wenigstens 6 Monate erstreckt.

9. Verfahren nach einem der Ansprüche 1 bis 8, **dadurch gekennzeichnet, dass** $\Delta T^{BA}$ mit zunehmender Betriebsdauer 70°C nicht überschreitet.

10. Verfahren nach einem der Ansprüche 1 bis 9, **dadurch gekennzeichnet, dass** $T^A$ über die gesamte Betriebsdauer im Bereich von 300 bis 400°C liegt.

11. Verfahren nach einem der Ansprüche 1 bis 10, **dadurch gekennzeichnet, dass** $T^B$ über die gesamte Betriebsdauer im Bereich von 305 bis 415°C liegt.

12. Verfahren nach einem der Ansprüche 1 bis 11, **dadurch gekennzeichnet, dass** sich das Katalysatorfestbett in den Reaktionsrohren eines zwei Temperaturzonen aufweisenden Rohrbündelreaktors befindet.

13. Verfahren nach einem der Ansprüche 1 bis 12, **dadurch gekennzeichnet, dass** der Langzeitbetrieb unterbrochen und das Katalysatorfestbett durch Durchleiten eines heißen Gasgemischs aus molekularem Sauerstoff und Inertgas durch das Katalysatorfestbett regeneriert und daran anschließend der Langzeitbetrieb fortgesetzt wird.

14. Verfahren nach einem der Ansprüche 1 bis 13, **dadurch gekennzeichnet, dass** das Reaktionsgaseingangsgemisch 2 bis 25 Vol.-% Propen enthält.

15. Verfahren nach einem der Ansprüche 1 bis 14, **dadurch gekennzeichnet, dass** das wenigstens eine Inertgas zu wenigstens 40% seines Volumens aus molekularem Stickstoff besteht.

16. Verfahren nach einem der Ansprüche 1 bis 15, **dadurch gekennzeichnet, dass** das Reaktionsgaseingangsgemisch den molekularen Sauerstoff und das Propen in einem molaren Verhältnis $O_2 : C_3H_6 \leq 3$ enthält.

17. Verfahren nach einem der Ansprüche 1 bis 16, **dadurch gekennzeichnet, dass** sich $\Delta T^{BA}$ mit zunehmender Betriebsdauer um wenigstens 5°C erhöht.

18. Verfahren nach einem der Ansprüche 1 bis 17, **dadurch gekennzeichnet, dass** die maximale Reaktionstemperatur in der Reaktionszone A, $T^{maxA}$, mit zunehmender Betriebsdauer 420°C nicht überschreitet.

19. Verfahren nach einem der Ansprüche 1 bis 18, **dadurch gekennzeichnet, dass** die maximale Reaktionstemperatur in der Reaktionszone A, $T^{maxA}$, $\geq 350°C$ beträgt.

20. Verfahren nach einem der Ansprüche 1 bis 19, **dadurch gekennzeichnet, dass** die Differenz zwischen der maximalen Reaktionstemperatur in der Temperaturzone A, $T^{maxA}$, und der Temperatur $T^A$ der Temperaturzone A, gebildet als $\Delta T^{HB}A = T^{maxA} - T^A$, 40 bis 90°C beträgt.

**Claims**

1. A process for long-term operation of a heterogeneously catalyzed partial gas phase oxidation of propene to acrolein, in which a reaction gas input mixture which comprises propene, molecular oxygen and at least one inert gas and comprises the molecular oxygen and the propene in a molar $O_2 : C_3H_6$ ratio of $\geq 1$ is conducted through a fixed catalyst bed whose active material is at least one multimetal oxide comprising the elements Mo, Fe and Bi, with the proviso that

- the fixed catalyst bed is arranged in two spatially successive temperature zones A, B,
- both the temperature $T^A$ of temperature zone A and the temperature $T^B$ of temperature zone B are a temperature in the temperature range from 280 to 420°C,
- the reaction gas input mixture flows through temperature zones A, B in the time sequence "first A" and "then B", temperature zone A extending up to a conversion $C^A$ of the propene present in the reaction gas input mixture in the range from 45 to 85 mol%, and the conversion of the propene increasing in temperature zone B to a value $C^B$ of $\geq 90$ mol%,
- in single pass of the reaction gas input mixture through the overall fixed catalyst bed, the selectivity of acrolein formation, based on propene converted, is $\geq 80$ mol%,
- the space velocity of propene present in the reaction gas input mixture on the fixed catalyst bed is $\geq 140$ 1 (STP) of propene/l of fixed catalyst bed.h,
- the temperatures $T^A$ and $T^B$ on completion of fresh charging of the fixed catalyst bed are such that the difference $\Delta T^{BA} = T^B - T^A > 0°C$,
- then, with increasing operating time, in order to counteract the reduction in the quality of the fixed catalyst bed, at least one of the two temperatures $T^A$, $T^B$ is increased, wherein
- the increasing of the at least one of the two temperatures $T^A$, $T^B$ is undertaken such that the difference $\Delta T^{BA}$ $T^B - T^A$ increases with increasing operating time.

2. The process according to claim 1, wherein, on completion of fresh charging of the fixed catalyst bed, $\Delta T^{BA} \geq 2°C$.

3. The process according to claim 1 or 2, wherein, on completion of fresh charging of the fixed catalyst bed, $\Delta T^{BA} \leq 50°C$.

4. The process according to any of claims 1 to 3, wherein, on completion of fresh charging of the fixed catalyst bed, the difference between the maximum reaction temperature in temperature zone A, $T^{maxA}$, and the maximum reaction temperature in temperature zone B, $T^{maxB}$, formed as $T^{maxA} - T^{maxB}$, is $\geq 0°C$ und $\leq 80°C$.

5. The process according to any of claims 1 to 4, wherein both $T^B$ and $T^A$ are increased in long-term operation.

6. The process according to claim 5, wherein, based on the same operating time in long-term operation, the increase in $T^B$ is 1.2 to 5 times the increase in $T^A$.

7. The process according to any of claims 1 to 6, wherein the difference between the maximum reaction temperature in temperature zone A, $T^{maxA}$, and the maximum reaction temperature in temperature zone B, $T^{maxB}$, formed as $T^{maxA}$ $T^{maxB}$, is reduced with increasing operating time, but does not become negative.

8. The process according to any of claims 1 to 7, wherein the operating time extends to at least 6 months.

9. The process according to any of claims 1 to 8, wherein $\Delta T^{BA}$ does not exceed 70°C with increasing operating time.

10. The process according to any of claims 1 to 9, wherein $T^A$ is within the range from 300 to 400°C over the entire operating time.

11. The process according to any of claims 1 to 10, wherein $T^B$ is within the range from 305 to 415°C over the entire operating time.

12. The process according to any of claims 1 to 11, wherein the fixed catalyst bed is present in the reaction tubes of a tube bundle reactor having two temperature zones.

13. The process according to any of claims 1 to 12, wherein the long-term operation is interrupted and the fixed catalyst bed is regenerated by passing a hot gas mixture of molecular oxygen and inert gas through the fixed catalyst bed, and then the long-term operation is continued.

14. The process according to any of claims 1 to 13, wherein the reaction gas input mixture comprises 2 to 25% by volume of propene.

15. The process according to any of claims 1 to 14, wherein the at least one inert gas consists of molecular nitrogen to an extent of at least 40% of its volume.

16. The process according to any of claims 1 to 15, wherein the reaction gas input mixture comprises the molecular oxygen and the propene in a molar $O_2 : C_3H_6$ ratio of $\leq 3$.

17. The process according to any of claims 1 to 16, wherein $\Delta T^{BA}$ increases by at least 5°C with increasing operating time.

18. The process according to any of claims 1 to 17, wherein the maximum reaction temperature in reaction zone A, $T^{maxA}$, does not exceed 420°C with increasing operating time.

19. The process according to any of claims 1 to 18, wherein the maximum reaction temperature in reaction zone A, $T^{maxA}$, is $\geq 350$°C.

20. The process according to any of claims 1 to 19, wherein the difference between the maximum reaction temperature in temperature zone A, $T^{maxA}$, and the temperature $T^A$ of temperature zone A, formed as $\Delta T^{HB}_A = T^{maxA} - T^A$, is 40 to 90°C.

**Revendications**

1. Procédé d'exploitation à long terme d'une oxydation partielle en phase gazeuse sous catalyse hétérogène de propène en acroléine, selon lequel un mélange gazeux réactionnel d'entrée contenant du propène, de l'oxygène moléculaire et au moins un gaz inerte, qui contient l'oxygène moléculaire et le propène en un rapport molaire $O_2:C_3H_6 \geq 1$, traverse un lit catalytique fixe dont la masse active est au moins un oxyde de plusieurs métaux contenant les éléments Mo, Fe et Bi, à condition que

   - le lit catalytique fixe soit agencé dans deux zones de température A, B successives dans l'espace,
   - aussi bien la température $T^A$ de la zone de température A que la température $T^B$ de la zone de température B soient une température dans la plage de température allant de 280 à 420 °C,
   - le mélange gazeux réactionnel d'entrée traverse les zones de température A, B selon la chronologie « tout d'abord A » et « puis B », la zone de température A s'étendant jusqu'à une conversion $U^A$ du propène contenu dans le mélange gazeux réactionnel d'entrée dans la plage allant de 45 à 85 % en moles et la conversion du propène augmentant dans la zone de température B à une valeur $U^B \geq 90$ % en moles,
   - lors du passage unique du mélange gazeux réactionnel d'entrée au travers de l'ensemble du lit catalytique fixe, la sélectivité pour la formation d'acroléine, par rapport au propène transformé, soit $\geq 80$ % en moles,
   - le chargement du lit catalytique fixe avec le propène contenu dans le mélange gazeux réactionnel d'entrée soit $\geq 140$ Nl de propène/l de lit catalytique fixe·h,
   - les températures $T^A$ et $T^B$ après le chargement frais du lit catalytique fixe soient telles que la différence $\Delta T^{BA} = T^B - T^A$ soit > 0 °C,
   - au moins une des deux températures $T^A$, $T^B$ soit ensuite augmentée lorsque la durée d'exploitation augmente afin de contrer la réduction de qualité du lit catalytique fixe, **caractérisé en ce que**
   - l'augmentation d'au moins une des deux températures $T^A$, $T^B$ est réalisée de sorte que la différence $\Delta T^{BA} = T^B - T^A$ augmente lorsque la durée d'exploitation augmente.

2. Procédé selon la revendication 1, **caractérisé en ce que** $\Delta T^{BA} \geq 2$ °C après le chargement frais du lit catalytique fixe.

3. Procédé selon la revendication 1 ou 2, **caractérisé en ce que** $\Delta T^{BA} \leq 50$ °C après le chargement frais du lit catalytique fixe.

4. Procédé selon l'une quelconque des revendications 1 à 3, **caractérisé en ce qu'**après le chargement frais du lit catalytique fixe, la différence entre la température de réaction maximale dans la zone de température A, $T^{maxA}$, et la température de réaction maximale dans la zone de température B, $T^{maxB}$, formée par $T^{maxA} - T^{maxB}$, est $\geq 0$ °C et $\leq 80$ °C.

5. Procédé selon l'une quelconque des revendications 1 à 4, **caractérisé en ce qu'**aussi bien $T^B$ que $T^A$ augmentent lors de l'exploitation à long terme.

**6.** Procédé selon la revendication 5, **caractérisé en ce que** l'augmentation de $T^B$ est de 1,2 à 5 fois l'augmentation de $T^A$ par rapport à la même durée d'exploitation en exploitation à long terme.

**7.** Procédé selon l'une quelconque des revendications 1 à 6, **caractérisé en ce que** la différence entre la température de réaction maximale dans la zone de température A, $T^{maxA}$, et la température de réaction maximale dans la zone de température B, $T^{maxB}$, formée par $T^{maxA} - T^{maxB}$, diminue lorsque la durée d'exploitation augmente, mais ne devient pas négative.

**8.** Procédé selon l'une quelconque des revendications 1 à 7, **caractérisé en ce que** la durée d'exploitation s'étend sur au moins 6 mois.

**9.** Procédé selon l'une quelconque des revendications 1 à 8, **caractérisé en ce que** $\Delta T^{BA}$ ne dépasse pas 70 °C lorsque la durée d'exploitation augmente.

**10.** Procédé selon l'une quelconque des revendications 1 à 9, **caractérisé en ce que** $T^A$ se situe dans la plage allant de 300 à 400 °C pendant l'ensemble de la durée d'exploitation.

**11.** Procédé selon l'une quelconque des revendications 1 à 10, **caractérisé en ce que** $T^B$ se situe dans la plage allant de 305 à 415 °C pendant l'ensemble de la durée d'exploitation.

**12.** Procédé selon l'une quelconque des revendications 1 à 11, **caractérisé en ce que** le lit catalytique fixe se trouve dans les tubes de réaction d'un réacteur à faisceau de tubes comprenant deux zones de température.

**13.** Procédé selon l'une quelconque des revendications 1 à 12, **caractérisé en ce que** l'exploitation à long terme est interrompue et le lit catalytique fixe est régénéré par passage d'un mélange gazeux chaud d'oxygène moléculaire et de gaz inerte dans le lit catalytique fixe, puis l'exploitation à long terme est poursuivie.

**14.** Procédé selon l'une quelconque des revendications 1 à 13, **caractérisé en ce que** le mélange gazeux réactionnel d'entrée contient 2 à 25 % en volume de propène.

**15.** Procédé selon l'une quelconque des revendications 1 à 14, **caractérisé en ce que** le ou les gaz inertes sont constitués d'azote moléculaire à hauteur d'au moins 40 % de leur volume.

**16.** Procédé selon l'une quelconque des revendications 1 à 15, **caractérisé en ce que** le mélange réactionnel gazeux d'entrée contient l'oxygène moléculaire et le propène en un rapport molaire $O_2$: $C_3H_6 \leq 3$.

**17.** Procédé selon l'une quelconque des revendications 1 à 16, **caractérisé en ce que** $\Delta T^{BA}$ augmente d'au moins 5 °C lorsque la durée d'exploitation augmente.

**18.** Procédé selon l'une quelconque des revendications 1 à 17, **caractérisé en ce que** la température de réaction maximale dans la zone de réaction A, $T^{maxA}$, ne dépasse pas 420 °C lorsque la durée d'exploitation augmente.

**19.** Procédé selon l'une quelconque des revendications 1 à 18, **caractérisé en ce que** la température de réaction maximale dans la zone de réaction A, $T^{maxA}$, est $\geq 350$ °C.

**20.** Procédé selon l'une quelconque des revendications 1 à 19, **caractérisé en ce que** la différence entre la température de réaction maximale dans la zone de température A, $T^{maxA}$ et la température $T^A$ de la zone de température A, formée par $\Delta T^{HB}_A = T^{maxA} - T^A$, est de 40 à 90 °C.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004085362 A
- EP 700714 A
- EP 1159244 A
- EP 1547994 A
- DE 19910508
- DE 19948523
- DE 19910506
- DE 19948241
- WO 2007082827 A
- WO 2004085370 A
- WO 2004085369 A
- WO 2004085363 A
- WO 2004085365 A
- WO 2004007064 A
- WO 2004085367 A
- DE 2830765 C
- DE 2513405 C
- US 3147084 A
- DE 2201528 A
- EP 383224 A
- DE 2903218 A
- WO 2005009608 A
- EP 1577001 A
- EP 1180508 A
- EP 1106598 A
- DE 102004025445 A
- DE 10351269 A
- DE 10350812 A
- EP 614872 A
- DE 102004008573 A
- WO 05082517 A
- DE 10232748 A
- WO 2004009525 A
- DE 19955176 A
- DE 19948523 A
- DE 10101695 A
- DE 19948248 A
- DE 19955168 A
- DE 102009047291 A
- DE 102007005606 A
- DE 10200840094 A
- WO 2008087116 A
- WO 2008087115 A
- WO 2007017431 A
- DE 102007004961 A
- DE 102008040093 A
- DE 102008042064
- DE 102008042061
- DE 102008042060 A
- WO 2005042459 A
- DE 10046957 A
- DE 10063162 A
- DE 3338380 C
- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- EP 293224 A
- WO 02062737 A
- DE 4023239 A
- DE 2909671 A
- EP 293859 A
- EP 714700 A
- DE 102008054586
- DE 102008040094 A
- DE 19910508 A
- DE 19910506 A
- DE 19948241 A
- WO 200408362 A
- EP 700893 A
- EP 1547944 A
- EP 468290 B
- DE 2201528 B
- EP 382098 A
- WO 0136364 A
- EP 873783 A
- EP 1270065 A
- US 7534339 B2
- WO 200713504 A
- WO 2007060036 A
- WO 2007042457 A
- WO 2006002713 A
- WO 2006002708 A
- WO 2006002703 A
- DE 10302715 A
- EP 982287 A
- EP 982289 A
- DE 19924532 A

- DE 10115277 A
- DE 19606877 A
- DE 19740252 A
- DE 19627847 A
- DE 10053086 A
- EP 982288 A

**In der Beschreibung aufgeführte Nicht-Patentliteratur**

- *Schriften Research Disclosure Nr. 497012,* 29. August 2005